# EUROPEAN PATENT APPLICATION

(11) **EP 4 566 654 A2**
(43) Date of publication of application: **11.06.2025**
(21) Application number: 25171349.1
(22) Date of filing: 16.10.2020
(51) Int. Cl.: A61N 1/04

(54) **ELECTROTRANSFER THERAPEUTIC DELIVERY DEVICE, SYSTEM AND METHOD**

(30) Priority: 18.10.2019 AU 2019903941; 27.03.2020 AU 2020900955
(62) Divisional of application: 20877908.2
(71) Applicant: NewSouth Innovations Pty Limited, Sydney, New South Wales 2052 (AU)
(72) Inventor: HOUSLEY, Gary David, Sydney, 2052 (AU); PINYON, Jeremy, Sydney, 2052 (AU); LOVELL, Nigel Hamilton, Sydney, 2052 (AU); CRAWFORD, Edward Norman, Sydney, 2052 (AU); AL ABED, Amr, Sydney, 2052 (AU); VON JONQUIERES, Georg, Sydney, 2052 (AU)
(74) Representative: Nederlandsch Octrooibureau

(57) **Abstract**

An needle electrode array includes a needle as a first electrode; a first concentric insulator sheathing the needle to a first distance from the needle tip, a concentric second electrode sheathing the first insulator to a distance from the first insulator's tipward end, and a second concentric insulator sheathing the second electrode to a distance from the second electrode's tipward end.

The needle and second electrode are formed of conductive material, with electrical connections to positive and negative terminals of a pulse delivery circuit.

The exposed length of the needle operates as first electrode and the exposed length of the second electrode as second electrode.

The lengths of the first and second electrodes and exposed length of the insulating portion therebetween determines electric field gradients generated adjacent the needle array when an electric pulse is applied to drive one electrode as anode and the other as cathode.

## Description

### Technical Field

The field of the invention is electric field stimulated cell transfection to deliver therapeutic molecules, in particular using a linear electrode array on a single probe. An application of the invention is for DNA electrotransfer into cells.

### Background

Electric field stimulated transfection of cells for delivery of therapeutics to the cells, or electrotransfer, is commonly known as electroporation. Electroporation is commonly achieved by injecting tissue with a therapeutic (for example, a solution of therapeutic molecules), inserting two separate probes into tissue, on either side of a target treatment area, one acting as a cathode and the other an anode, and to pass electric current through the tissue between the two probes. This causes temporary disruption to the cell membrane to allow penetration of the therapeutic into the cell, also known as transfection. This is a known method for delivery of gene therapy.

When the DNA encodes a gene for a protein, or part thereof, this electric-field based delivery of DNA to cells may be referred to as 'Gene Electrotransfer' (GET). Electroporation of DNA into cells is a routine laboratory process. Electroporation / GET is also a recognised means for achieving gene expression in tissue, either in situ, or in vivo, although the efficiency of gene expression in tissues following DNA electroporation is generally low.

Traditional electroporation requires high voltage pulses, and as such specialised equipment and clinical settings. This can limit the accessibility of such therapies.

Development of techniques for stimulating transfection within a target area adjacent a contiguous electrode array, is described in the inventors' previous patent applications, publication nos. WO2011/006204, WO2014/201511 and WO2016/205895 the disclosure of which provides background to the present disclosure, and may be referred to for a better understanding of the inventors' close field electroporation techniques. This previous work by the inventors has shown that surprising electrotransfer efficiency has been achieved using a "close field" electrotransfer technique using single probe linear arrays of electrodes, such that cells are transfected in tissue adjacent the linear array. For example, the electrode array comprises two or more elongate electrodes, linearly aligned, and spaced apart at discrete positions along the length of a single probe. The electrodes are contiguous to a substrate array structure, such that no tissue will lie directly between the electrodes. Electric field gradients are generated in tissue adjacent the array, when an electroporation pulse is applied, the shape of the electric field and gradients of the electric field being controlled by the configuration of the linear elongate electrodes.

In WO2016/205895 the inventors disclose a system where the area within which electronically stimulated cell transfection occurs is controlled by controlling gradients within the generated electric field based on the electrode array configuration and pulse sequence applied to generate the electric field. The system of WO2016/205895 illustrates a practical application of the discovery, by the inventors, of improved cell transfection within an area of tissue subject to steeper electric field potential gradients, and application of this discovery to control the shape of the electric field to generate contours in the electric field to target regions for cell transfection adjacent the array. The variables for controlling the electric field shape included the array configuration and stimulation pulse parameters. Using controlled electric field shaping (an electric field effect) has enabled improved efficacy of cell transfection at lower cumulative charge than previously known with open field electroporation.

This close field electrotransfer technique has demonstrated advantages in requiring lower cumulative charge than conventional electroporation techniques. Another advantage is ability to predictably control the shape and size of the region of tissue where cell transfection occurs based on a combination of linear electrode array configuration and electroporation pulse parameters, which control the electric field gradients of the generated electric field. However, there is a desire to further improve electrotransfer techniques.

### Summary of the Invention

According to a first aspect there is provided an electrotransfer system comprising: at least one probe, each probe comprising: a probe body; a needle electrode array extending from the probe body configured as a needle to be inserted into tissue to be treated; and a capacitive discharge circuit connected to the needle electrode array comprising capacitive charge storage configured to store a quantum of charge, and a switch actuatable to cause discharge of the stored quantum of charge through the needle electrode array, the needle electrode array incorporating at least two electrodes, each electrode having a surface area substantially circumferential to the needle and exposed to directly contact tissue into which the needle electrode array is inserted, with an insulating section between neighbouring electrodes to form a contiguous linear array structure, the exposed surface area of each electrode being at a different distance from the needle electrode array tip, each electrode connected to the capacitive discharge circuit for driving as an anode or cathode during discharge of the quantum of charge, and wherein electrode length and length of the insulating section between neighbouring electrodes are configured to produce a target electric field shape in tissue adjacent to the array during discharge of the quantum of charge via the array; and a charging station having a DC power supply module, output terminals connectable to a probe to form electric contact to the capacitive discharge circuit, and charging control circuitry to control charging of the capacitive discharge circuit of the connected probe.

In an embodiment the capacitive discharge circuit is housed in the probe body.

Some embodiments of the electrotransfer system further comprise a switch actuator carried on the probe body configured to enable actuation of the capacitive discharge circuit switch.

In some embodiments the charging station includes a probe mounting to support the probe during charging.

In some embodiments the electrodes are relatively elongate, wherein the electrode diameter is less than the electrode length. In some embodiments the electrodes have a length of 1mm or less and an electrode diameter less than the electrode length.

In some embodiments capacitive discharge circuit parameters are matched to resistivity parameters of a therapeutic solution for electrotransfer to minimise total charge delivered and optimise the discharge time constant for electrotransfer. For example, capacitance of the capacitive discharge circuit can be chosen based on a predicted total resistivity during discharge and a target accumulative charge, and the predicted total resistivity is based on the therapeutic solution resistivity. For example, the target for the mating can be to achieve a discharge time constant of between 20 µs and 2 s.

In some embodiments the probe body further comprises a therapeutic reservoir and a lumen through the needle in fluid communication with the therapeutic reservoir to hold a volume of fluid, and a therapeutic delivery actuator operable to cause the fluid to be forced from the therapeutic reservoir and through the lumen and be discharged from the needle. In some embodiments the therapeutic delivery actuator is further configured to activate the switch. In some embodiments the probe is packaged with a therapeutic solution stored within the reservoir. In alternative embodiments the reservoir is filled with a therapeutic solution prior to charging the capacitive discharge circuit.

In some embodiments the needle electrode comprises: a first needle which also acts as a first electrode; a first concentric insulator sheathing the first needle to a predetermined first distance (L1) from the needle tip, a concentric second electrode sheathing the first concentric insulator to a distance (L2) from a tipward end of the first concentric insulator, and a second concentric insulator sheathing the second electrode to a distance (L3) from a tipward end of the second electrode, wherein the first needle and second concentric electrode are formed of conductive material and are electrically connected to positive and negative terminals of the capacitive discharge circuit.

In some embodiments the exposed length L1 of the first needle operates as a first electrode of a linear array, and the exposed length L3 of the second concentric electrode acts as the second electrode of a linear array. The respective lengths of the first and second electrodes, and exposed length of the first insulating portion therebetween can determine the pattern of electric field gradients generated adjacent the needle array when an electric pulse is applied to drive one electrode as an anode and the other electrode as a cathode.

According to another aspect there is provided an electrotransfer system probe comprising: a probe body; a needle electrode array extending from the probe body configured as a needle to be inserted into tissue to be treated; and a capacitive discharge circuit connected to the needle electrode array comprising capacitive charge storage configured to store a quantum of charge, and a switch actuatable to cause discharge of the stored quantum of charge through the needle electrode array, wherein the needle electrode array incorporates at least two electrodes, each electrode having a surface area substantially circumferential to the needle and exposed to directly contact tissue into which the needle electrode array is inserted, with an insulating section between neighbouring electrodes to form a contiguous linear array structure, the exposed surface area of each electrode being at a different distance from the needle electrode array tip, each electrode connected to the capacitive discharge circuit for driving as an anode or cathode during discharge of the quantum of charge, and wherein electrode length and length of the insulating section between neighbouring electrodes are configured to produce a target electric field shape in tissue adjacent to the array during discharge of the quantum of charge via the array.

In an embodiment the needle electrode comprises: a first needle of which a length of the needle proximate the needle tip provides the first electrode; a first concentric insulator sheathing the first needle to a predetermined first distance (L1) from the needle tip, a conductive sheath sheathing the first concentric insulator to a distance (L2) from a tipward end of the first concentric insulator and forming the second electrode, and a second concentric insulator sheathing the second electrode to a distance (L3) from a tipward end of the second electrode, wherein the first needle and second concentric electrode are formed of conductive material and are electrically connected to positive and negative terminals of the capacitive discharge circuit. The respective lengths of the first and second electrodes, and exposed length of the first insulating portion therebetween determines the pattern of electric field gradients generated adjacent the needle array when an electric pulse is applied to drive one electrode as an anode and the other electrode as a cathode.

Some embodiments of the probe further comprise a therapeutic reservoir and a lumen through the needle array in fluid communication with the therapeutic reservoir to hold a volume of fluid, and a therapeutic delivery actuator operable to cause the fluid to be forced from the therapeutic reservoir and through the lumen and be discharged from the needle. In some embodiments the therapeutic delivery actuator is further configured to activate the switch.

In some embodiments the probe body has a two part form, wherein a first part incorporating the reservoir and therapeutic delivery actuator is provided by a syringe connectable to a second part comprising an electrotransfer module having a housing adapted to engage with the syringe, and support the needle electrode array projecting outward from the housing, wherein the capacitive discharge circuit is housed within the housing is connected to the needle electrode array, and a fluid communication path is formed through the housing between the syringe reservoir and the lumen of the needle electrode. For example, the electrotransfer module housing can be Luer-lock compatible to engage with a convention Luer syringe

According to another aspect there is provided a needle electrode array comprising: a first needle which also acts as a first electrode; a first concentric insulator sheathing the first needle to a predetermined first distance (L1) from the needle tip, a concentric second electrode sheathing the first concentric insulator to a distance (L2) from a tipward end of the first concentric insulator, and a second concentric insulator sheathing the second electrode to a distance (L3) from a tipward end of the second electrode, wherein the first needle and second concentric electrode are formed of conductive material and are electrically connected to positive and negative terminals of a pulse delivery circuit.

In some embodiments the exposed length L1 of the first needle operates as a first electrode of a linear array, and the exposed length L3 of the second concentric electrode acts as the second electrode of a linear array. In some embodiments the respective lengths of the first and second electrodes, and exposed length of the first insulating portion therebetween determines the pattern of electric field gradients generated adjacent the needle array when an electric pulse is applied to drive one electrode as an anode and the other electrode as a cathode.

In some embodiments the needle tip is bevelled.

In some embodiment of the needle electrode the first needle has a hollow lumen through which therapeutic solution can be delivered.

In some embodiments the therapeutic solution is delivered to tissue from an aperture in the tip of the first needle.

In some embodiments the needle array includes apertures in the array distal from the tip whereby therapeutic solution is delivered to tissue adjacent the needle array.

Another aspect provides a method of performing gene electrotransfer using a system as described above comprising the steps of: charging the probe; inserting the needle array into target tissue; delivering a therapeutic solution to the target tissue; actuating capacitive discharge; and removing the needle array form the tissue.

### Brief Description of the Drawings

Figure 1 is a representative diagram of an embodiment of a close field capacitive discharge electrotransfer system.
Figure 2 is a block diagram of an example of a charging unit for an embodiment of the system.
Figure 3 is an example of a capacitive charging circuit for an embodiment of the system.
Figure 4 is an example of the measured potential for a capacitive discharge.
Figure 5 is another example of a circuit diagram for a charging unit for an embodiment of the system.
Figure 6 comparatively illustrates a typical electroporation pulse profile and a capacitive discharge pulse profile.
Figures 7a and 7b illustrate results of a first (in vitro) study comparing expression of DNA encoding (transfection) using a prototype probe stimulated by sequences of square wave and exponentially decaying pulses.
Figure 8 illustrates results of a second study investigating the feasibility of cell transfection using a single capacitive discharge pulse.
Figure 9a illustrates bioluminescence imaging of a mouse at 24 hours post DNA electrotransfer in the encircled hindlimb regions.
Figure 9b & 9c are graphs showing peak total light flux emitted from each mouse leg at each time point post electrotransfer, showing peak total light flux emitted from each mouse leg sampled at time points up to 500 days post electrotransfer, evidencing maintenance of effects from the electrotransfer long term.
Figure 10 illustrates some effects of manipulating the gap between anode and cathode of the electrode array.
Figure 11 illustrates an example of an embodiment of a probe tip.
Figure 12 illustrates an example of an embodiment of a needle array.
Figure 13a shows a model of the electric potential in the medium around the DNA delivery probe when 240 V is applied across the electrodes as for the peak voltage evoked in the field adjacent to the probe during discharge of an integrated capacitor.
Figure 13b shows a model of the electric field strength around the DNA delivery probe when 240 V is transiently applied across the electrodes.
Figure 14 shows another example of an embodiment of a probe tip.
Figures 15a and 15b illustrate a conceptual rendering of insertion of a probe into a charging station.
Figure 16 illustrates a conceptual example of an embodiment of an embodiment of a probe configured as a syringe device and a compatible charging station.
Figure 17 is an image demonstrating the utility of a prototype SCD-BaDGE *in vivo* application, the image showing expression of light activatable ChrimsonR channelrhodopsin ion channel protein - TD tomato fusion reporter protein in mouse soleus muscle fibres.
Figure 18 illustrates an example of an embodiment of an electrotransfer module connectable to a syringe, and a charging unit.
Figures 19a and 19b show an example of an embodiment of an electrotransfer module and compatible syringe, shown as separate components in Figure 19a and connected in figure 19b.
Figure 20 shows an exploded view of an embodiment of an electrotransfer module and a compatible syringe.
Figure 21a illustrates and embodiment of an electrotransfer module connected to a syringe, providing a more detailed view of the electrotransfer module components.
Figure 21b illustrates an embodiment of an electrotransfer module connected to a low dead-space type syringe, and showing detail of the electrotransfer module components.
Figure 21c illustrates the components of an embodiment of the electrotransfer module in detail.
Figure 22 illustrates an embodiment of an electrotransfer module connected to a syringe, being placed in a module to charge the capacitive discharge circuit.
Figure 23 illustrates an example of an inoculation process, for delivery of a DNA vaccine, using an embodiment of the electrotransfer system;
Figure 24a illustrates a step of therapeutic delivery via the syringe and electrotransfer module.
Figure 24b illustrates a step of stimulating the tissue for electotransfer using an electric field generated in the tissue surrounding the needle array by the capacitive discharge.
Figure 25 is a photograph of a prototype embodiment of an electrotransfer module, showing the housing of one of the electrotransfer modules opened to show in interior components.
Figure 26a is a set of images showing the comparative cell transfection results for electrotransfer of DNA using a 100V single pulse with a cochlear implant electrode array with the individual cochlear electrodes grouped as an anode array and a cathode array (in tandem configuration), and concentric needle type arrays using single pulses of 100V, 40V, and 10V.
Figure 26b is an ANOVA graph of the results illustrated in figure 26a.
Figure 26c: Electric potential map (hemisphere) of BaDGE concentric stainless steel electrode array

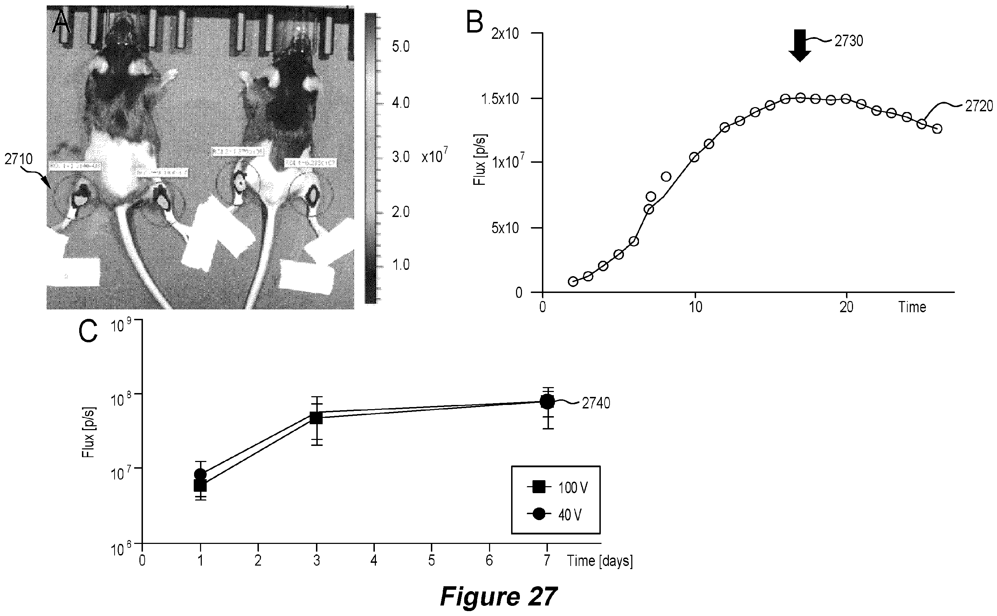

Figure 27 shows temporal tracking results of an embodiment of electrotransfer mediated gene expression. (A) Representative bioluminescence image demonstrating the spread of luciferase activity three days following intramuscular electrotransfer mediated luciferase gene (Luc) transfer to the mouse gastrocnemius muscle. (B) Representative trace of the emitted photons per second in a ROI placed over a hind limb, measured in one-minute intervals following i.p. luciferin injection. (C) Time course graphing bioluminescence measurements at peak (red arrow in B) one, three and seven days following BaDGE^{®} mediated transfer of the luciferase encoding plasmid.
Figures 28a-c are photographs of an example of a wired needle electrode embodiment.
Figure 29a-b illustrates a prototype test embodiment for high throughput needle electrode DNA electrotransfer in cell suspensions for autologous gene therapy and in vitro cell transfection.
Figure 30 shows images of HEK239 cells showing expression of an mCherry reporter plasmid DNA against the background of cells (DAPI nuclear stain), achieved using in vitro electrotransfer via the concentric stainless steel DNA electrotransfer electrode configuration.
Figure 31 illustrates another example of the electrotransfer system being utilised in in vitro cell therapies applicable to high throughput multi-well cell transfection with plasmid DNA via the DNA delivery probe.

### Detailed Description

One aspect disclosed herein is a device capable of high efficiency DNA electrotransfer into cells via a single capacitive discharge.

The principle of this system relates to the storage of a quantum of charge on a capacitor which is then discharged through an electrode array configured to produce a electric field having electric field potential gradients focussed through a region by the array configuration and of sufficient in strength for efficient electrotransfer of DNA into cells. This DNA, or related ribonucleic acid molecules or indeed other charged molecules, upon entering the cells, can affect changes in biological function.

In some embodiments this may be discharge of a single capacitor.

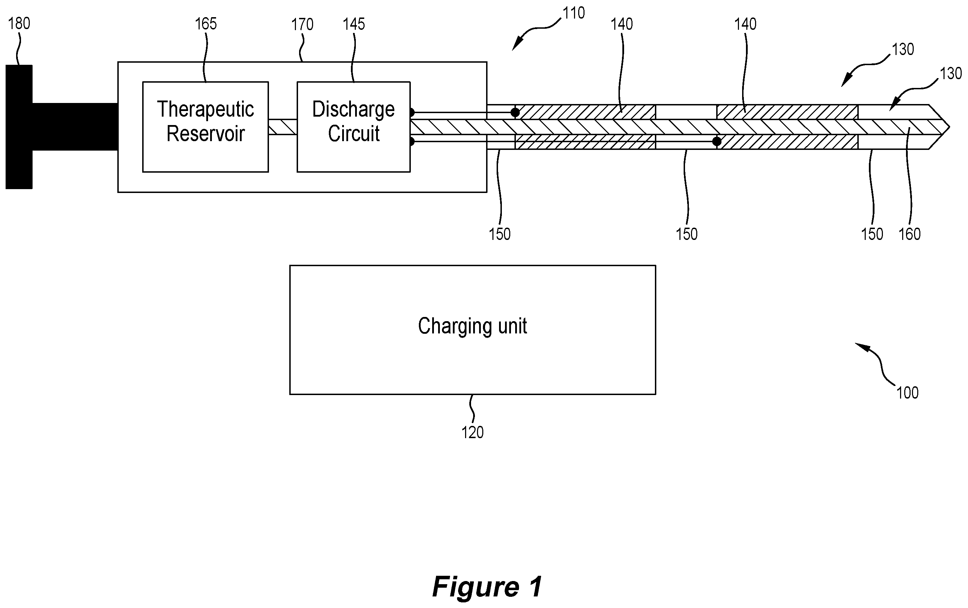

Figure 1 shows an example of an electrotransfer system 100 having at least one probe 110, and a charging station 120. Each probe 110 comprises a probe body 170, a needle electrode array 130 and a discharge circuit 145. In a most simple embodiment the discharge circuit includes a capacitor and a switch. The needle electrode array 130 extends from the probe body 170 and is configured as a needle to be inserted into tissue to be treated.

The needle electrode array 130 incorporates at least two electrodes 140, each electrode having a surface area either wholly or partially circumferential to the needle and exposed to directly contact tissue into which the needle electrode array is inserted, with an insulating section 150 between neighbouring electrodes 140. This forms a contiguous linear array structure, with the exposed surface area of each electrode at a different distance from the needle electrode array tip.

The capacitive discharge circuit 145 is connected to the needle electrode array and configured to store a quantum of charge. The circuit includes a switch actuatable to cause discharge of the stored quantum of charge through the needle electrode array 130. Each electrode 140 is connected to the capacitive discharge circuit for driving as an anode or cathode during discharge of the quantum of charge. The electrode length and length of the insulating section between neighbouring electrodes are configured to produce a target electric field shape in tissue adjacent to the array during discharge of the quantum of charge via the array. The combination of elongate electrodes, arranged in a line with a gap between, cause an electric field to be generated adjacent to the electrode array. The gap between the electrodes focusses the electric field in tissue in the region proximate the gap. The width of the gap controls the rate of change in the electric field over distance and thus the electric field intensity in the region proximate the gap, defining the shape of the electric field. Research by the inventors has shown that high electric field intensity, caused by the rate of change in the voltage potential due to the described linear array geometry, can stimulate electrotransfer using lower stimulation voltage and cumulative charge than conventional open field electroporation. Thus, enabling effective electrotransfer using a single pulse. The electric potential gradient can be varied by adjusting the length of the gap between the electrodes. Effectively focussing the electric field. There can also be some influence by varying electrode length. The combination of the electrode array geometry and stimulation pulse controls the size and shape of the region of the electrotransfer.

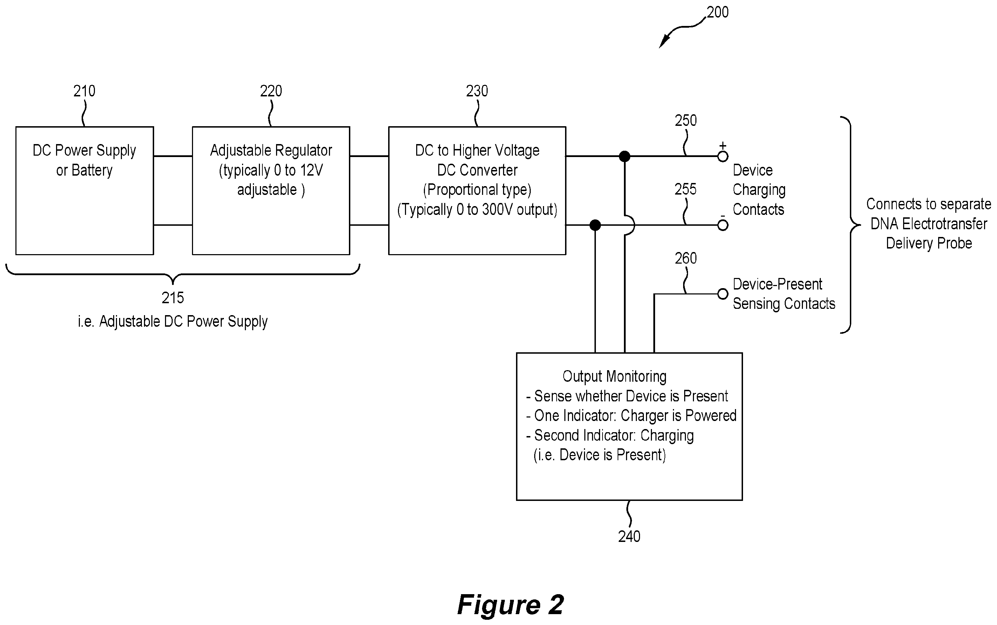

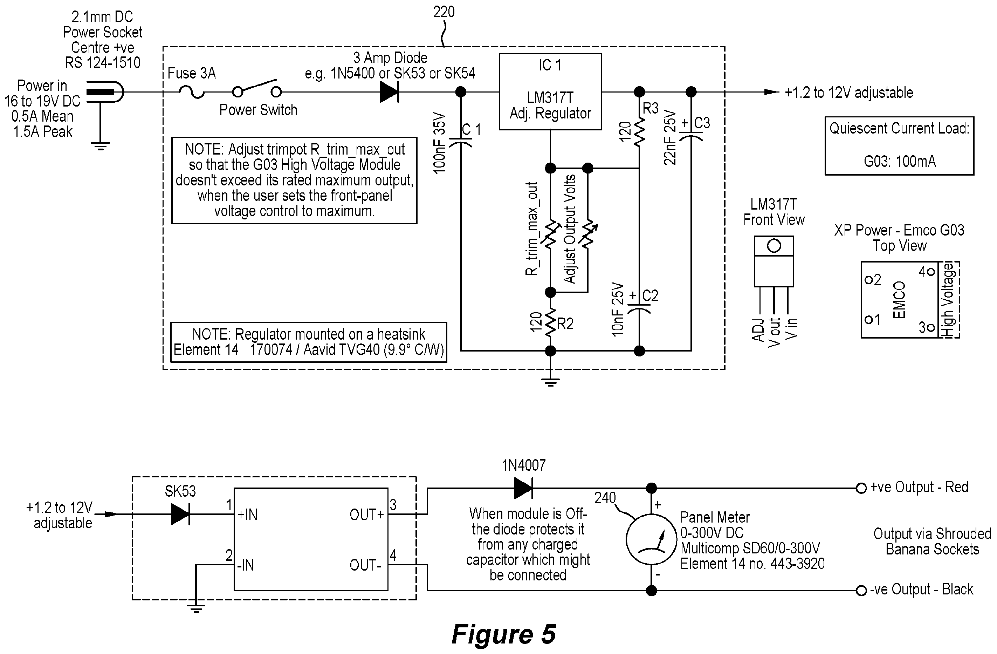

The charging station 120 has a DC power supply module, output terminals connectable to a probe 110 to form electric contact to the capacitive discharge circuit, and charging control circuitry to control charging of the capacitive discharge circuit of the connected probe. Figure 2 is a block diagram of an embodiment of a charging station 120, 200. The DC power supply 210 may be battery powered or a mains powered DC power supply. Some embodiments use an adjustable DC power supply 215, including both a DC power supply source 210 and an adjustable regulator 220 to enable a user, or automated controller, to adjust the output voltage. The charging station can include a DC-DC voltage converter 230. The charging station may also include an output monitoring module 240 configured to determine whether a probe is connected to the charger, and the status of the connected probe and capacitive discharge circuit. The monitoring module may also be configured to control the adjustable DC power supply to disconnect the power supply once a predetermined quantum of charge is stored in the capacitive discharge circuit. The station may include a sensor contact 260 to detect presence of a probe at the charger. The monitoring module 240 may also output status signals, for example using visual signal (i.e. LEDs) or audio signals. For example, the electronics of the charging station may incorporate elements for determining safe charging of the needle delivery probes, and an indicator of successful charging, and a timer to model slow leak of the capacitor with a Green/Red LED or an sudatory prompt or tone to indicate if too much time has passed for efficient DNA electrotransfer. Figure 5 illustrates circuit diagram for an example of the charging station.

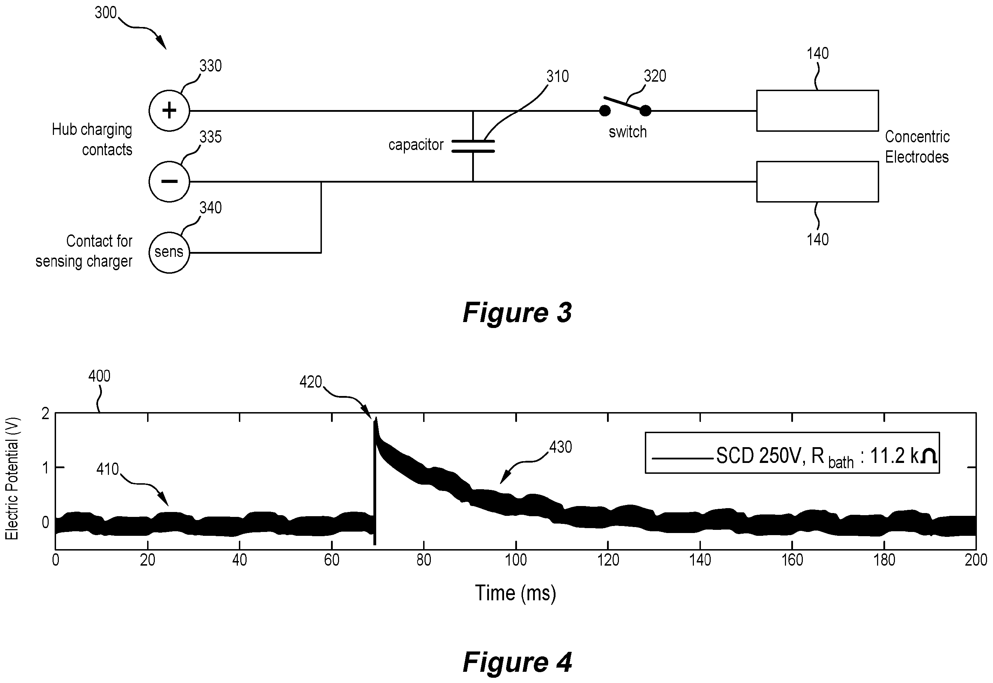

Figure 3 is an example of a capacitive discharge circuit 300 for an embodiment of the system. The circuit includes capacitive charge storage, in the example a single capacitor 310, a switch 320, and connections to the electrodes 140 of the needle electrode array. The circuit can also include charging contacts 330, 335 to establish an electrical connection to the charging station 200, for charging the capacitive charge storage with a quantum of charge. The switch is actuatable to cause discharge of the stored quantum of charge through the electrodes 140 of the needle electrode array 130. Each electrode 140 is connected to the capacitive discharge circuit, to be driven as an anode or cathode during discharge of the quantum of charge. Figure 4 shows an example of the electric potential 400 measured in the medium adjacent to the anode during discharge of the capacitive circuit, 410 shows the potential at zero prior to discharge (open circuit conditions up to around 70ms), prior to discharge of the capacitor. 420 Shows the rapid rise in voltage at the sampling point medium close to the electrode array, to a maximum voltage peak of around 2V, at around 70ms as the switch is closed and discharge is initiated, and the exponentially decaying potential 430 as the capacitor discharges.

In an embodiment the capacitive discharge circuit is housed in the probe body, thus to be able to deliver an electric pulse (the capacitive discharge) without needing to be connected to an external power supply and pulse generator. The capacitive storage may be a single capacitor. Alternatively, the capacitive storage may include more than one capacitor. In some embodiments the capacitive discharge circuit may be external to the probe body. In additional embodiments other charge storage media such as supercapacitors and batteries may be used instead of the capacitor. All such alternatives are considered within the scope of the present system. For example, for applications such as treatment of the brain, a priority may be to minimise the size of the probe to enable high precision and control for insertion, in such an embodiment the capacitive discharge circuit may be housed separate to the probe body, connected by wires. This will still provide the advantage of providing electrotransfer treatment without the need to connect to a mains powered pulse generator. Thus, equipment footprint in the clinical space may be reduced. Risk of electrical interference or electrocution are also reduced by not requiring a pulse generator. Examples of further embodiments are discussed below.

As mentioned above electrotransfer-based delivery of DNA to cells is commonly known as electroporation. When the DNA encodes a gene for a protein, this electric-field based delivery of DNA to cells may be referred to as 'Gene Electrotransfer' (GET). Electroporation of DNA into cells is a routine laboratory process. Electroporation / GET is also a recognised means for achieving gene expression in tissue, either in situ, or in vivo, although the efficiency of gene expression in tissues following DNA electroporation is generally low. Two related factors affecting GET are electric field strength (change in voltage over distance) and duration. The creation of a voltage gradient within tissue suitable for GET requires the flow of current between electrodes. This is achieved using an electroporator device which delivers current pulses between two or more electrodes to generate GET compatible electric fields (typically 10 - 100 V/cm), with typical durations of hundreds of microseconds to hundreds of milliseconds, With multiple iterations of these electric pulses within one or more pulse trains. A single electric pulse is generally understood to be inefficient in achieving GET. The instrumentation required for conventional GET therefore involves a device for generating trains of electric pulses of controlled voltage or current amplitude, duration and frequency, connected to electrodes inserted into the tissue. Such instrumentation is both costly and bulky.

There are evident advantages to GET if the electrodes can be independent of the electroporator controller, particularly with regard to the electrode geometry, where a wired connection to an electroporator controller, or an integrated electroporator microcontroller imposes constraints on tissue targets in the context of targeted delivery of DNA to various body regions.

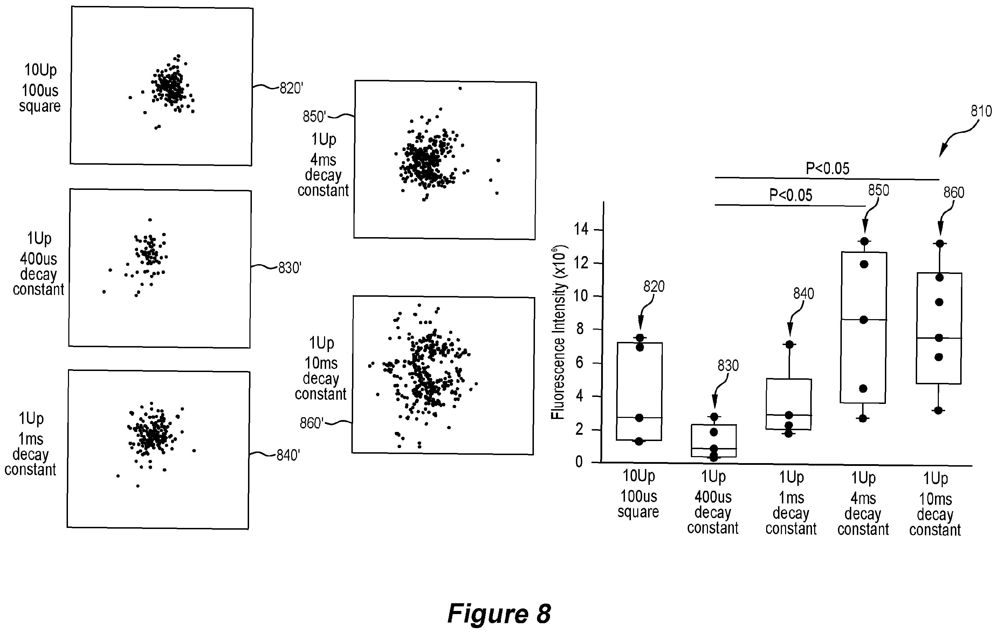

The inventors have discovered how to achieve efficient GET using a single electric pulse which does not require microprocessor-based control of the pulse parameters. This is achieved by separating the source of the charge from the control of the discharge, where the latter occurs via discharge of a capacitor. The source of the charge is a DC power supply which is used to charge the capacitor and can then be de-coupled from the capacitor circuit. In using the discharge of capacitor for GET, the inventors found that the discharge time of the capacitor was a critical factor - explained further below with reference to Figure 8. The capacitor time constant (tau), depends upon the size of the capacitor (C) and the resistance of the circuit (R), thus Tau = R.C. It should be appreciated that during discharge the total resistance in the circuit includes the resistance in the target tissue (influence by both the tissue and DNA carrier solution) and any circuit resistance. In this disclosure, we show that the combination of the very small current requirement for GET when delivered via a linear array of electrodes, in a 'close-field' electroporation configuration (two or more elongate electrodes arranged one after the other in a line along a single probe with electrodes at one end to be driven as an anode or anode array and electrodes at the other end as a cathode or cathode array), augmented by control of the resistivity around the electrode array using a high resistivity DNA carrier solution, when matched to a particular capacitor range, enables efficient GET. We show enablement of single capacitive discharge GET, where an optimal (minimal) charge delivery is identified based on the unanticipated relationship of the time constant (tau) of the discharge to the GET efficiency. Where Q = C.V., and it is desirable to limit the total charge delivered Q, to minimize potential tissue damage, we show that single capacitive discharge GET can achieve equivalent efficiency of gene expression to conventional multi-pulse electroporation based on Q delivered. In some embodiments total circuit resistivity R during discharge can be predicted, and the capacitance C chosen based on the target charge delivery Q. The total circuit resistivity R during discharge can be predicted based on the resistivity of the carrier solution and typical tissue resistivity ranges or test results for the type of target tissue (i.e. muscle tissue, fatty tissue, mammary tissue). This may also be predicted through testing of resistivity of tissue after injection with the carrier solution. The carrier solution can have a significant effect on the resistivity in the tissue, some carrier solutions may render the influence of the inherent resistance of the target tissue negligible. For example, resistivity of a high resistivity carrier solution may be in the range of 2 kOhms to 100 kOhms, the measured resistivity value for a 19% sucrose solution with a typical 2µg/µl DNA concentration is around 10 kOhms. The required cumulative charge Q required for DNA electrotransfer may be determined through prior testing, and thus be known. This may be a range indicating minimum cumulative charge to achieve electrotransfer and maximum cumulative charge to avoid tissue damage, a target cumulative charge value may be chosen within this range. Based on the resistivity and charge values the required capacitance for the discharge circuit can be calculated, and, if necessary, additional resistance to be included in the discharge circuitry encapsulated in the electroporation module. Thus, the capacity discharge circuit parameters can be matched to resistivity of the therapeutic solution. It should be appreciated that different types of carrier solutions can have different resistivity and thus the circuit capacitance chosen matched to the intended carrier solution.

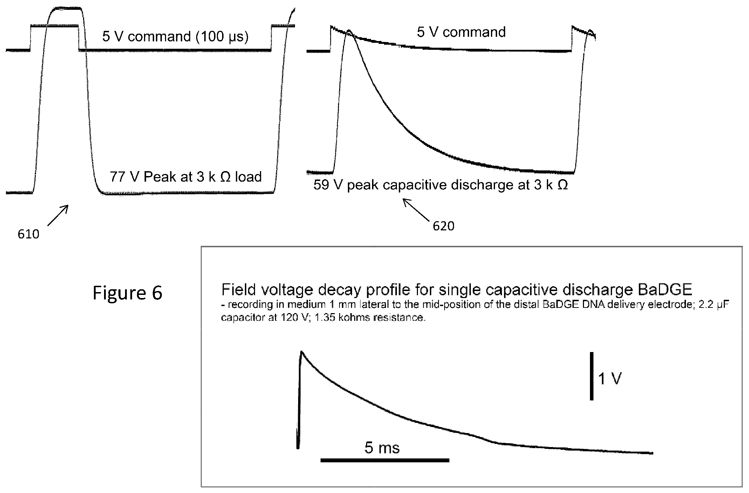

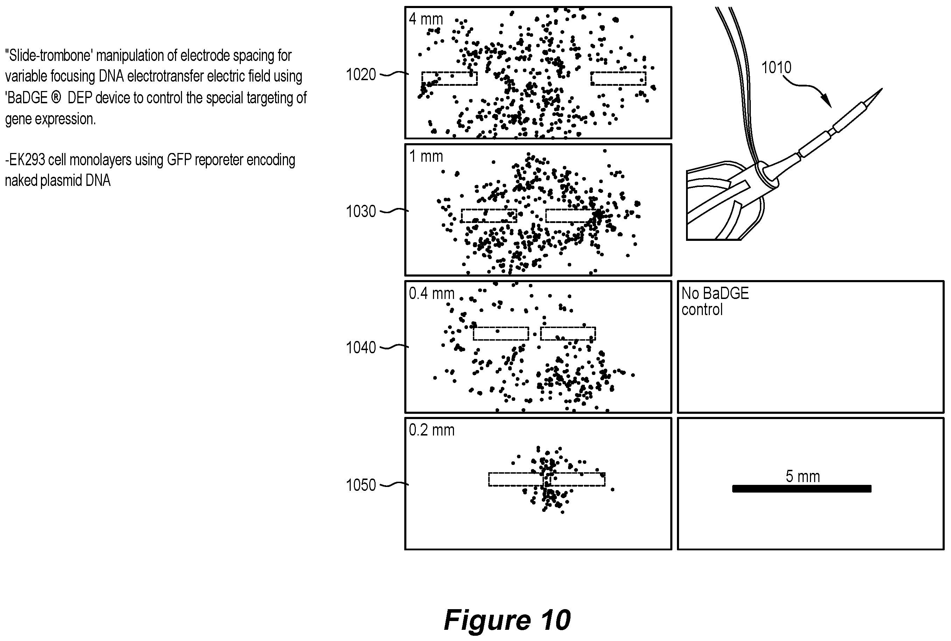

The data underlying the single capacitive discharge gene electrotransfer (SCDGET) invention consists of a series of *in vitro* studies using a human embryonic kidney (HEK293) cell monolayer model. We have previously used this model to develop the BaDGE - Bionic array Directed Gene Electrotransfer (BaDGET). Here a prototype BaDGE DNA delivery (1010 in Figure 10) probe was connected to either a conventional constant current power supply under computer control to delivery a specified square wave pulse train, or to deliver a pulse train, or single pulses, which followed a single exponential decay, reflecting the time constant for discharge of a capacitor. Figure 6 shows a comparison between a conventional square electroporation pulse and a modelled capacitive discharge pulse. In Figure 6 the left pane 610 shows a unipolar square wave pulse generated using a Digitimer DS5 analogue-controlled constant current supply (100 µs on, 400 µs off), and in the right pane 620 a modelled capacitive discharge pulse 100 µs time constant. These pulse parameters were used with the BaDGE prototype probe for delivery pf reporter DNA into HEK293 cell monolayers, modelling delivery to tissue.

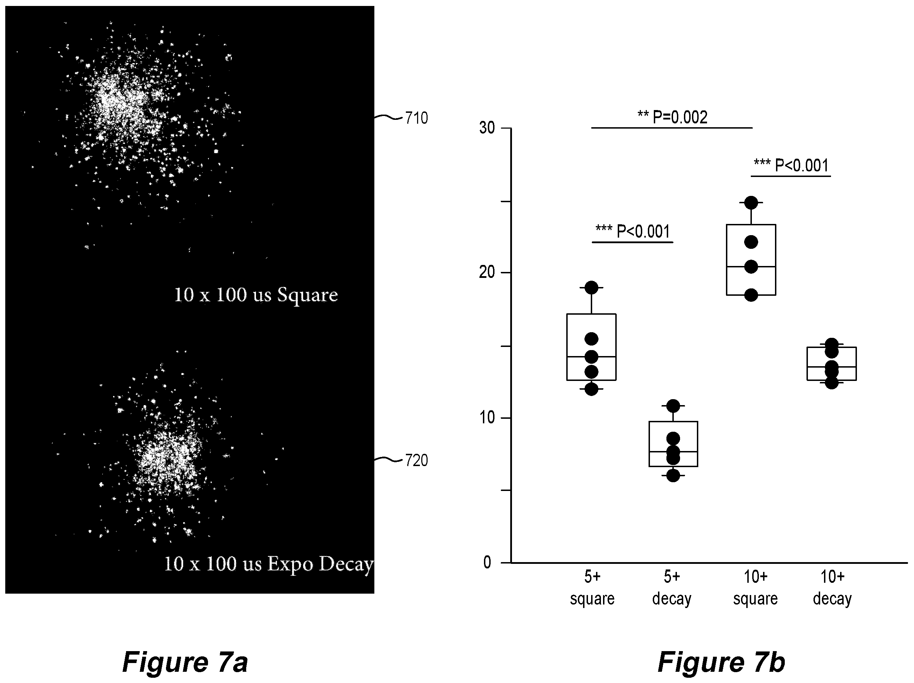

Figures 7a & 7b show data comparing expression of DNA encoding an mCherry fluorescent reporter protein across a series of these pulse trains. This study used a prototype electrode array (as illustrated 1010 in Figure 10) to compare results of electrotransfer using square wave unipolar 710 vs exponential decay 720 function in human embryonic kidney (HEK293) cells with CMVp-mCherry plasmid fluorescence reporter (2 µg / µl). The fluorescence shown in figure 7a was imaged 3 days after gene electrotransfer, the top pane 710 showing the transfection outcome using the square wave and the lower pane 720 showing the transfection outcome using the exponentially decaying pulse. In the results graph 7b, 5+ *square* represents 5 x 100 µs square wave constant current pulses (50 mA; 400 µs interpulse interval), *5+ decay* represents 5 x exponentially decaying (Capacitive) pulses, 100 µs time constant, 500 µs interpulse interval, *10+ square* represents 10 x 100 µs square wave pulses; *10+ decay* represents 10 x 100 µs decay time constant pulses. Ranked ANOVA with Holm-Sidak multiple post-hoc comparisons (* indicates P < 0.05; ranked ANOVA, post-hoc pairwise comparisons).

The results illustrated in Figures 7a and 7b show that using a 100 µs time constant (tau), a train of ten capacitor-like discharges at 120 V peak achieved equivalent expression to 5 square wave pulses of 50 mA amplitude x 100 µs duration (400 µs inter-pulse interval). The capacitive discharge was not as efficient at 10 x 50 mA x 100 µs square wave pulses, while the capacitive discharge total charge delivery (Q) was ~ equivalent. These data showed that a rapid decaying discharge achieved significant GET with minimal total charge delivery. However, multiple discharge pulses were required, which typically necessitates microprocessor-based control of the pulse train.

Figures 8 shows the results of a second study, which pursued the question of whether single capacitor discharge GET could be achieved if the time constant was increased. The study compared reference gene expression arising from 10 x 50 mA x 100 µs square wave pulses (as above, 5 replicates of HEK293 cell coverslips), against single capacitive discharge pulses with time constants of 400 µs, 1 ms, 4 ms and 10 ms; charged to 120 V which was equivalent to the square wave pulse parameter (based on 50 mA current amplitude across ~ 2 kohm resistance). These data showed that the GET efficiency was highly dependent upon the capacitor time constant, where a single pulse with a 1 ms time constant (equivalent Q to the 10 x 100 µs square wave pulses) produced statistically equivalent gene expression (integrated mCherry fluorescence) as the reference square wave pulse train. In addition, there was a non-linear dependency on the time constant for capacitor discharge, where a 4 ms time constant produced significantly greater gene expression than the reference level, and the 1 ms decay time constant treatment, and further extending the time constant (to 10 ms) achieved no further efficiency gain, despite the increase on charge delivery (Q). In summary, these two experiments showed the proof of concept that single capacitive discharge gene electrotransfer could be achieved with comparable efficiency (expression magnitude relative to charge delivered) to conventional square wave electroporation pulses.

The results illustrated in Figure 8 show that the prototype BaDGE^{®} Capacitive Discharge Gene Electrotransfer (BCDGET) (single pulse) is at least as efficient as a conventional unipolar square wave constant current pulse train for eliciting gene expression. Graph 810 shows data for mCherry reporter fluorescence from coverslips of HEK293 cells (n = 5 per treatment), where the plasmid DNA encoding this reporter was placed on the cells and the prototype BaDGE^{®} DNA delivery probe was used to electrotransfer the DNA to the cells. The coverslips were imaged after 3 days in tissue culture. Data shows equivalency between our reference 820. 820' of 10 x 100 µs square wave constant current pulses (~ 50 mA per pulse - designated as'100Up 100us square' and 1 single capacitive discharge with a 400 microsecond time constant 830, 830' (e.g. '1 up 400us decay constant'), 1 single capacitive discharge with a 1 millisecond time constant 840, 840' (e.g. '1 up 1ms decay constant'), 1 single capacitive discharge with a 4 millisecond time constant 850, 850' (e.g. '1 up 4ms decay constant'), and 1 single capacitive discharge with a 10 millisecond time constant 860, 860' (e.g. '1 up 10ms decay constant'). Single capacitive discharges with longer time constants showed significantly greater gene electrotransfer. The lower panels in Figure 8 show examples of imaging of mCherry reporter fluorescence of HEK293 cells on coverslips using the different charge delivery protocols (400 µs time constant = ~200 nF; 1 ms = ~500 nF, 4 ms = ~2 µF, 10 ms = ~5 µF (* indicates P < 0.05; ranked ANOVA, post-hoc pairwise comparisons)).

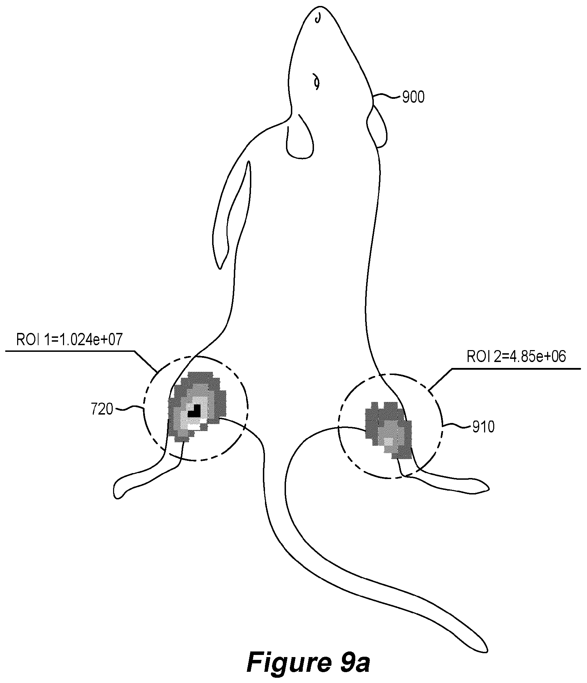

To validate these findings in a first *in vivo* proof of concept study, a mouse 900 hindlimb model was used (Figure 9a). Under isoflurane anaesthesia, DNA encoding the luciferase gene (1 µg / µl; 50 µl) was delivered to the right gastrocnemius muscle 910 using a prototype BaDGE^{®} DNA delivery probe (1010 in Figure 10), driven by a conventional constant current pulse train (5 x 4 ms pulses, 16 ms inter-pulse interval, ~ 60V recorded as driving a 50 mA current amplitude for each pulse). The plasmid DNA incorporated a hybrid cytomegalovirus - chicken β-actin - rabbit β globin gene splice acceptor sequence (CAG promoter) and was 6604 bp in size). The left gastrocnemius muscle 920 was treated with a single capacitive discharge gene electrotransfer, where the BaDGE^{®} DNA delivery probe was connected to a 2.2 µF capacitor charged to 120 V. This delivered a single capacitive discharge with a time constant of 4 ms (based on a 2 kohm resistance model), where the total charge delivered was ~50% of that used for the reference 5 x 4 ms squarewave pulse train delivered to the right hindlimb target. The expression was analysed 24 hours later using the SPECTRUM CT bioluminescence imaging platform, within 30 min of intraperitoneal injection of the bioluminescence substrate luciferin. The images show stronger expression (bioluminescence) in the left hindlimb - arising from the single capacitive discharge GET than from the conventional squarewave pulse train GET.

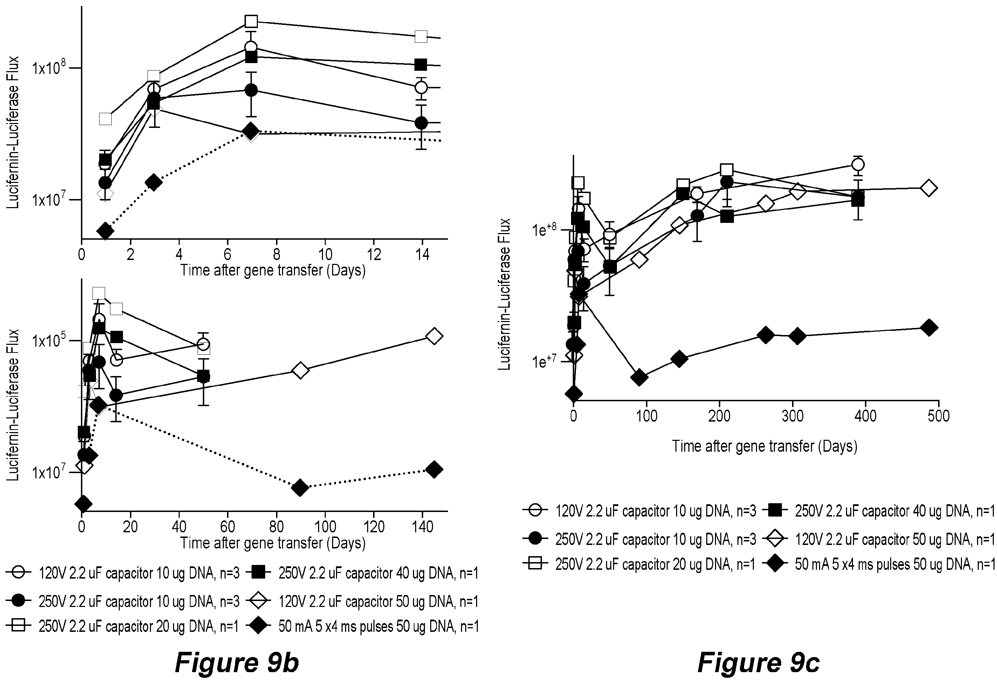

Figure 9a illustrates efficacy of the *in vivo* proof of concept for a prototype Single Capacitive Discharge Gene Electrotransfer (SCDGET) device. Figure 9 shows spectrum CT bioluminescence imaging of a mouse (ref. ms187, 250519) at 24 hours post DNA electrotransfer, under isoflurane anaesthesia within 30 min of i.p. injection of luciferin substrate to elicit photo emission arising from expression of recombinant luciferase by the hindlimb muscles. The emissions 920 from the left hindlimb result from DNA (1 µg / µl) electrotransfer to gastrocnemius skeletal muscle target utilised the BaDGE^{®} DNA delivery probe, coupled to a 2.2 µF capacitor, charged to 120 V. The right leg 910 utilized the same probe driven with 5 x 4 ms pulses (16 ms interpulse interval), (50 mA current via ~ 60 V driving voltage) using a constant current power supply (Digitimer DS5 analogue control), via software & D - A control. To get the image shown in Figure 9a, the mouse was anaesthetised with isoflurane and then injected (i.p. - intraperitoneal) with luciferin - the substrate for the luciferase enzyme that was expressed. This substrate was taken up by the muscle cells, and in the presence of luciferase expression, photons of light were produced that passed out through the skin and were counted by a very sensitive camera. This recording of emitted light reflects strength of expression of the recombinant luciferase protein. The readout was 24 hours following expression, and is shown overlaid on the image of the mouse hind limbs, as intensity coded light emission. This image shows that the SCDGET utilised ~ 50% of the charge delivery of the conventional square wave pulse train protocol and elicited ~ twice the bioluminescence signal.

Measurement of the bioluminescence signal (light flux) are shown graphed in figure 9b for mouse ref. 187 as well as additional experiments in both legs of four additional mice. It can be observed that mouse 187 (the diamond traces) has multiple reads at 1, 3, 7 90, & 150 days. The other mice have readouts at 1,3,7, 14 & 50 days. Figure 9b illustrates comparative longer term results for Bionic array-Directed Gene Electrotransfer (BaDGE) of plasmid DNA (pMK175.CAGp-Luciferase) into the mouse calf muscle using a single discharge from a 2.2 uF capacitor integrated into the BaDGE DNA delivery probe. Peak total light flux emitted from each mouse leg was measured at each time point following intraperperotoneal injection of D-Luciferin potassium salt (15 mg per kg of body weight). In this study, mice underwent BaDGE at day 0 using a plasmid encoding a luciferase gene. DNA was suspended in 10% sucrose solution pH balanced with sodium hydroxide. For mouse legs that received 10 µg of DNA; 20 µl was delivered at 0.5 µg/ul, for those that received 20, 40 or 50 µg of DNA this was delivered at 1.0 µg/ul, in a volume of 20, 40 or 50 µl. Diamond symbols represent left and right legs of mouse ref. 187; circles are the mean ± s.e.m. data from mice ref. 119, 120, 121. Squares are data from mouse ref. 118, with two DNA payloads.

Figure 9c shows Bioluminescence readout of Luciferase reporter gene expression (LUC) following BaDGE^{®} - mediated DNA electrotransfer. Image shows mouse #187 with peak expression maintained out to 486 days and similar sustained expression at 389 days for mice # 118, 119, 120 & 121. . Imaging at peak of luciferin substrate-mediated bioluminescence (i.p. administration) dark diamond represents 4 x 5 ms squarewave pulses (5 ms) vs open diamond showing 1 x 2.2 µF capacitive discharge from 120 V (both 1 µg/µl pMK175-CAGp-Luc plasmid DNA). Graph shows data from 5 mice in total (n = 3 for 250 V (black circle) or 120 V (open circle), 2.2 µF, 10 µg in 20 µl); open square is 20 µg at 1µg/µl DNA 250 V 2.2 µF; black square is 40 µl DNA at 1 µg/µl at 250 V, 2.2 uF single capacitive discharge. At the time of filing data up to 486 days is the longest term data available, due to the timing of the tests, however given that sustained effects are observed up to 486 days, this provides a positive indication of continued ongoing effect.

These studies demonstrated a prototype device, where a capacitor integrated into the BaDGE^{®} DNA delivery probe could be charged via a charging station (voltage-regulated power supply - typically taking < 1s to charge) and then the DNA delivery probe, unencumbered by a connection to a controlled power supply, was used for the DNA electrotransfer, by simply shorting the circuit (a switch). Separate tests showed that once the capacitor was charged, its voltage was maintained for at least 30 minutes.

Embodiments of the system disclosed stem from the discovery that linear arrays of electrode elements configured into a DNA delivery probe enable high efficiency electrotransfer of naked DNA into target tissues. Charge transfer needed to achieve gene expression in vivo is orders of magnitude lower than conventional electroporation systems; due to focused compression of the local electric field in the vicinity of the linear probe.

Another discovery applied in embodiments of the system is that the electrode configuration can control the range of the transfection region relative to a linear electrode array. Figure 10 illustrates an example of a prototype linear electrode array probe 1010. This prototype electrode array comprises two elongate tubular platinum-iridium (Pt-Ir) electrodes, connected to a capacitive discharge circuit (or a pulse generator) by insulated Pt-Ir wires, the Pt-Ir electrodes overlaid on an internal needle with an insulation layer between, insulating the electrodes from the internal needle and each other. One electrode is driven and an anode and the other as a cathode during pulse delivery. The electric field gradient adjacent the middle of the array has the steepest electric field potential gradient, and experimental results have shown that this region is where the most cells are transfected. The field shape is due to a combination of the linearly arranged elongate anode and cathode, and the respective dimensions of these conductive elements.

The elongate nature of the anode and cathode contribute to control of the generated electric field shape, and hence the transfection region. Altering the width of the gap between the elongate anode and cathode alters the range of the transfection area orthogonal to the array due to varying the compression of the electric field and rate of change of electric potential with distance adjacent the array proximate the gap. By altering the gap, the electric field shape and hence the transfection area can be expanded or contracted. In the example shown, increasing the gap between the electrodes expands the electric field and transfection area, whereas reducing the gap confined the field. It should be noted that this field shape control or vectoring is applicable for up to a gap of around 5 mm between electrodes for the pulse parameter used in this example. Increasing this gap may be feasible in conjunction with a corresponding increase in current levels, however due to potential negative side effects caused by higher current levels using further sets of electrodes may be preferable to enable treatment of a larger region. Controlling the radial spread of the electric field can also be referred to as vectoring.

Figure 10 illustrates some effects of manipulating the gap between anode and cathode, these results being achieved using prototype probes. Figure 10 images 1020 to 1050 illustrate the expansion and contraction of the transfection region with manipulation of the gap between the anode and cathode of the linear electrode array, in the examples of Figure 10 probes of the type shown 1010 having linear, two electrode arrays with different spacing between the electrodes were used. The effect of manipulation of the electric field - achieved by decreasing the separation between two platinum-iridium electrodes (2 mm length x 0.35 mm diameter on an insulated scaffold - as shown in the upper right image), for each example array configuration the electrodes were driven using a train of electric pulses (constant current, 100 µs x 10 pulses, 400 µs pulse separation, 10 mA +ve phase). The data shows the spatial control of electrotransfer of plasmid DNA (a green fluorescent reporter protein - GFP - under a cytomegalovirus (CMV) promoter) delivered to monolayers of Human Embryonic Kidney (HEK) 293 cells, using an isotonic polysaccharose carrier. The DNA and the electrotransfer array were placed over the cells on a coverslip. The pulses were delivered and then the coverslip returned to culture for 48 hours prior to imaging. The highest electric field strength is around the null point between the two electrodes. As the electrodes separate, the electric field sufficient to DNA electrotransfer expands, until an optimum dispersion and cell density of expression of the recombinant DNA expression cassette is obtained (between 1 - 4 mm electrode separation).

In embodiments of the capacitive discharge electrotransfer probe disclosed herein, a combination of the electrode array configuration (notably the electrode length and gap between the electrodes or groups of electrodes driven using the same polarity) and capacitive discharge characteristics control the region within which cell transfection can occur. Thus, the array configuration and discharge circuit can be designed to achieve a predictable treatment outcome. Further, the tissue and therapeutic solution injected into the region surrounding the linear array form part of the discharge circuit, therefore affect the characteristics of the capacitive discharge, and so the capacitive discharge circuit may be designed incorporating the therapeutic solution and/or tissue resistance in an equivalent circuit to model the discharge characteristics and electric field. Thus, probes may be designed tailored for specific therapeutic solutions and target tissue. For example, the capacitor characteristics for the discharge circuit may be matched to the resistivity of the therapeutic solution to provide a target initial voltage potential, cumulative charge delivery and pulse decay profile. The physical array configuration - length of electrodes and gap therebetween - can also be modelled for specific applications.

It is envisaged for a practical embodiment that SCDGET would be integrated into a disposable SCDGET-DNA delivery probe, including pre-packaged DNA in carrier solution, within a syringe. The SCDGET-probe would be charged just prior to use, by contact with a SCDGET charging station, the DNA would be delivered via the syringe and needle and the circuit closed to discharge the capacitor, which instantaneously achieves the GET. An indicator (e.g. LED) on the charging station would confirm that the SCDGET-probe was charged and indicate how long the charge was maintained in the probe, based on the discharge characteristics of a comparable capacitor circuit within the charging station. As noted, the probe charging time is less than a second. The charge maintenance time is tens of minutes. The physical size of the capacitors of the range suitable for optimized SCDGET (typically 1 - 5 µF, but can be other values) is in the low mm³ range, and unit costs are a few cents. This therefore lends itself to the high throughput DNA GET applications such as DNA vaccines, where the SCDGET charging station could be battery powered for field work, the DNA probes would be pre-loaded with DNA and one-use disposable items.

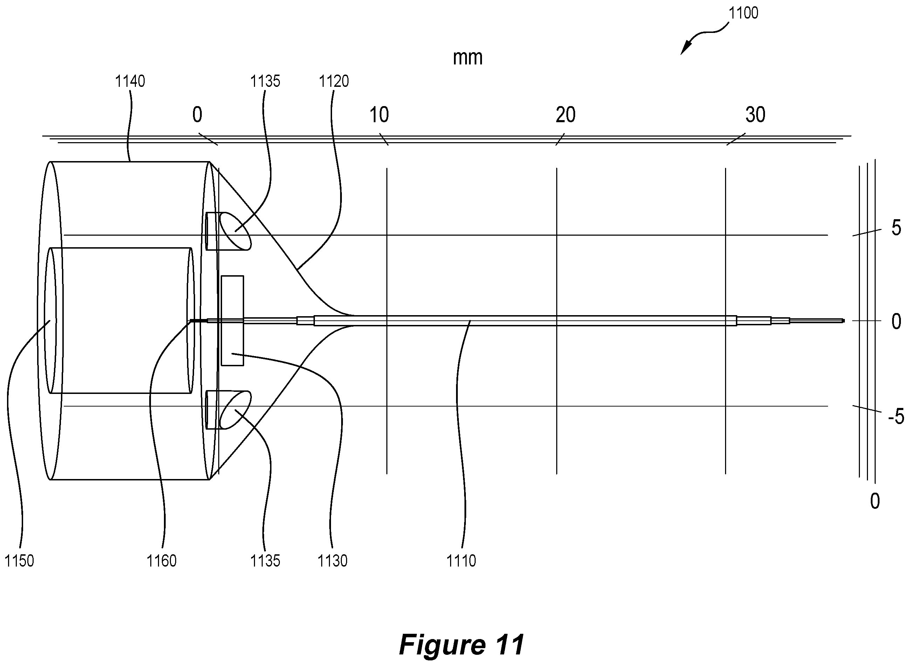

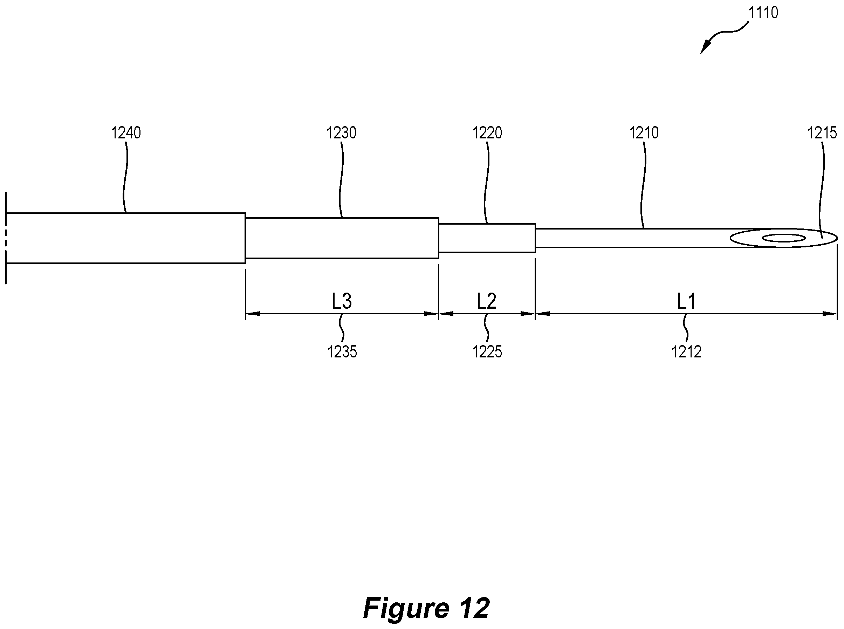

Figure 11 illustrates an example of a prototype probe tip 1100, comprising a needle array 1110, needle hub 1120, and body 1140 (of which only a portion is shown in the figure). The hub 1120 may house the capacitive discharge circuit 1130 and electrical connection to the electrodes of the needle array. In the embodiment shown in Figure 12 the hub 1120 also includes two ports 1135 to enable electrical connection to a charging station for charging the capacitive discharge circuit. This embodiment also illustrates a therapeutic reservoir in fluid communication with a central lumen through the needle array 1110 to enable the probe to be preloaded with therapeutic solution for delivery prior to application of the electrotransfer pulse. For example, the probe tip may be integrated into a conventional syringe structure with a plunger to force fluid from the reservoir through the lumen. In such an embodiment the plunger may also be configured to actuate a switch of the capacitive discharge circuit to cause capacitive discharge. For example, the switch may be actuated at the end of travel of the plunger.

The distal (tip) portion of the needle array 1110 is shown in more detail in Figure 12 and illustrates a first needle 1210 which also acts as a first electrode (in this embodiment having a bevelled tip 1215 and a central lumen for therapeutic delivery), a first concentric insulator 1220 sheathing the first needle to a predetermined distance (L1) 1212, from the tip 1215, a concentric second electrode 1230 sheathing the first concentric insulator to a distance (L2) 1235 from the tipward end of the first concentric insulator, and a second concentric insulator 1240 sheathing the second electrode 1230 to a distance (L3) 1235 from the tipward end of the second electrode 1230. The first needle 1210 and second concentric electrode are formed of conductive material and are electrically connected to positive and negative terminals of the capacitive discharge circuit 1130. The exposed length 1212 of the first needle operates as a first electrode of a linear array, and the exposed length 1235 of the second concentric electrode 1230 acts as the second electrode of a linear array. The respective lengths L1 1212 and L3 1235 of the first and second electrodes, and exposed length 1225 of the first insulating portion 1220 therebetween determines the pattern of electric field gradients generated adjacent the needle array when an electric pulse is applied to drive one electrode as an anode and the other electrode as a cathode.

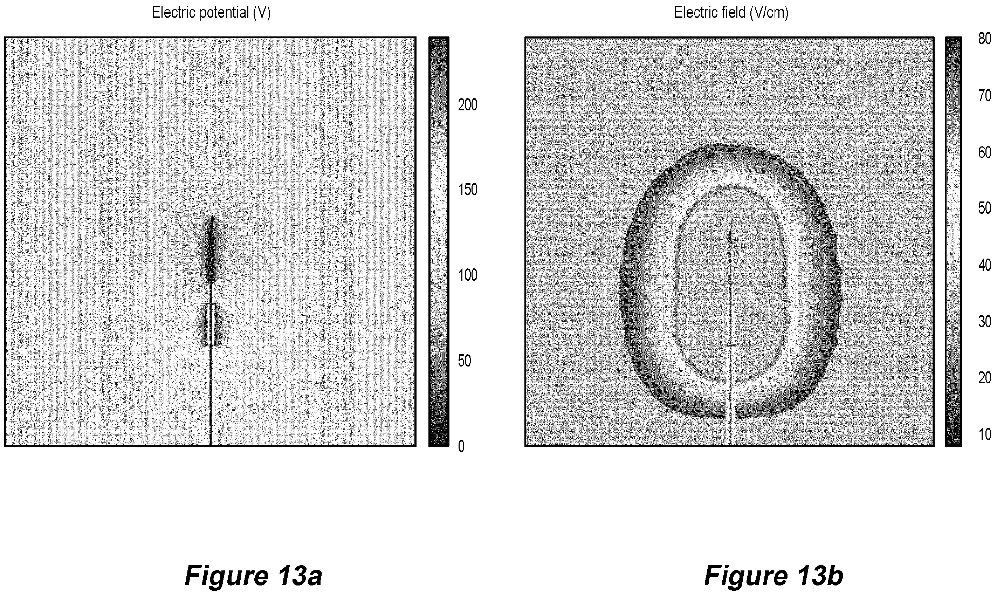

Figure 13a shows a model of the electric potential in the medium around the DNA delivery probe when 240 V is applied across the electrodes as for the peak voltage evoked in the field adjacent to the probe during discharge of an integrated capacitor. Figure 13b shows a model of the electric field strength around the DNA delivery probe when 240 V is transiently applied across the electrodes.

An embodiment of the needle array is constructed using two concentric needles, where the internal needle is insulated starting a pre-set distance from the tip, the uninsulated tip region being the 1^{st} electrode, and the outer needle being insulated from a set distance back from its tip. There also being a gap between the position of the second (shorter) needle and the insulated region of the inner needle (that distance of insulated coating on the inner needle before its conductive tip being equivalent to the separation between electrode elements shown in figure 10 for the "slide trombone" effect.

Both needles are inserted into a custom designed needle "hub". This hub incorporates circuitry that couples to the proximal ends of the electrodes - which again are conductive. The hub can incorporate holes for the insertion of the charging station pins, and a switch-plate which closes the discharge circuit for the integrated capacitor. In an embodiment the switch-plate is activated when the plunger of the syringe barrel is fully depressed, or at a predefined point of travel.

An example of prototype development involves use of a 34 G needle with a bevel tip (36 mm long) and a 28 G outer needle (27 mm long) square cut - thin walled needle to act as the circumferential second electrode. In an embodiment the outer needle may also be bevelled to facilitate the transition in needle diameter from the thinner inner needle to the outer needle. Alternative embodiments of the needle array may be fabricated using deposition (for example sputtering, printing, etc) of conductive and insulation layers to form the electrodes.

In an alternative embodiment the needle array has a closed tip and ports formed along the length of the needle array (distal form the tip) for delivery of fluid to the adjacent tissue. An advantage of this embodiment is flexibility in the location of the conductive regions of the inner and outer needles and optimum delivery of the DNA and carrier solution in proximity to the electrodes, thereby achieving optimal control of the capacitive discharge time.

In some embodiment the needle electrode may include a plurality of electrodes along the array. For example, to allow for multiple ports for delivery of therapeutic solution from between electrodes. In some embodiments the needle electrode may include multiple electrodes ganged together or individually connected to one or more capacitive discharge circuits. In an embodiment where multiple discharge circuits are used these may be configured to be actuated to discharge at different times, to alter the shape of the electric filed during treatment. For example, with different capacitive discharge circuits actuated at different positions of travel of a syringe plunger. Different discharge circuits may also have different discharge characteristics.

In an example the probe could have a dozen electrodes and half a dozen capacitors connected to pairs of electrodes along the linear array. In another embodiment the elution of the DNA solution may also come from a port or multiple ports between electrode pairs and not necessarily from the tip of the needle.

In some embodiments the needle array may not include a lumen for therapeutic delivery. For example, the therapeutic may be delivered using another syringe. For some clinical applications or treatments, it may be desirable to use separate devices for delivery of therapeutics and delivery of electric pulses. The lumen may be dispensed with in order to minimise diameter of the needle array for physiological reasons, such as to produce very fine needle arrays for use within the brain or for young paediatric patients. In these embodiments therapeutic agent may be injected or otherwise applied to the target tissue prior to placement of the DNA electrotransfer probe. Alternatively, the needle array may be coated with a hydrogel coating containing the therapeutic so this is delivered to the tissue form the eternal surface of the needle array. In another embodiment the lumen may have a plurality of outlet ports to dispense the therapeutic along its length.

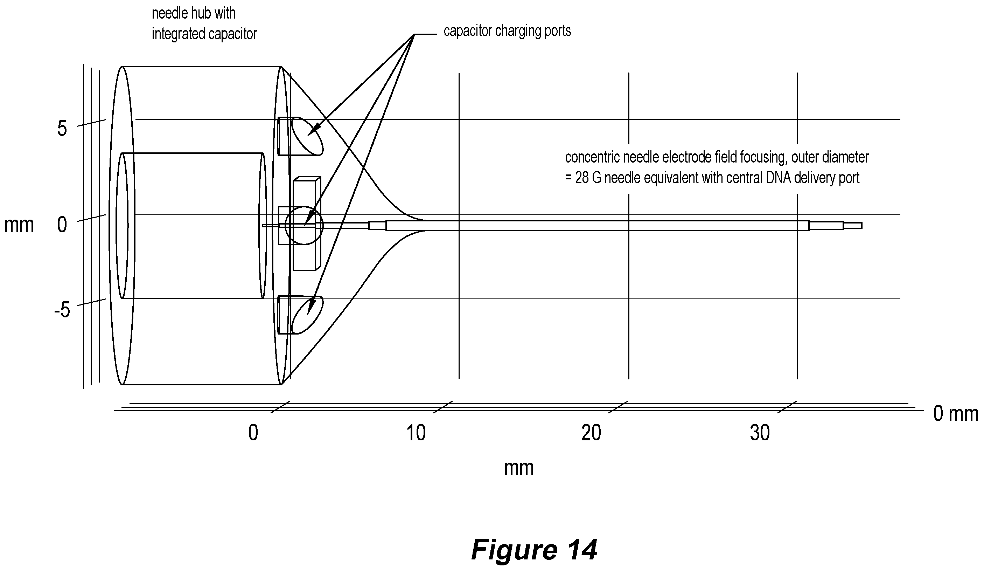

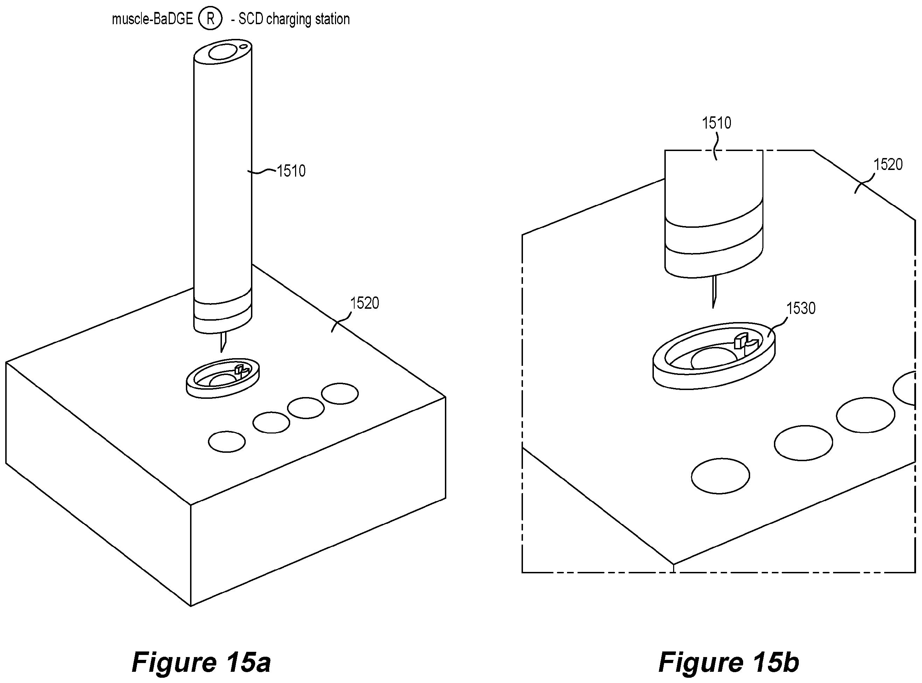

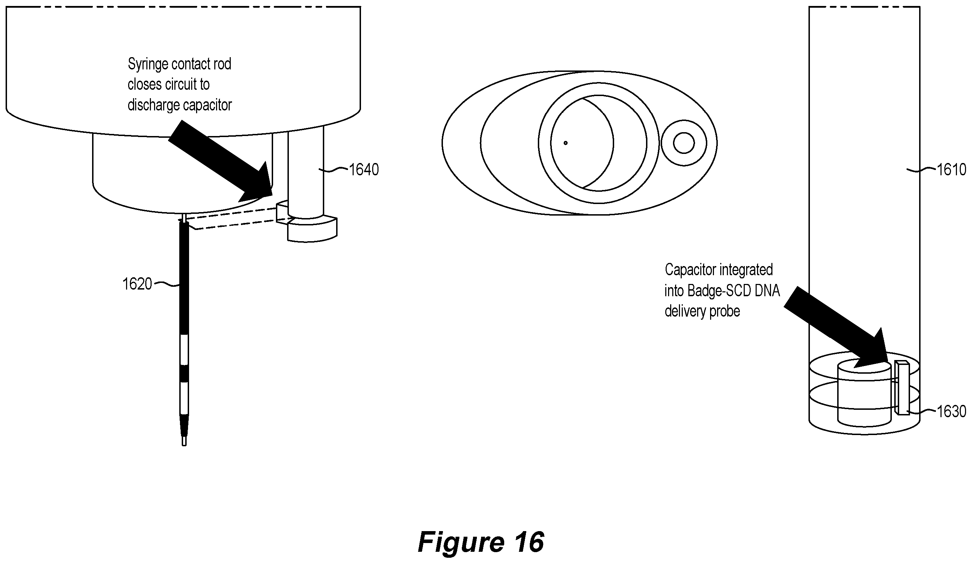

Figure 14 shows another embodiment of a probe tip, similar to that shown in figure 11. However, in this example the hub includes an additional port to enable contact with a sensor of the charging station, to check whether the probe is connected to the charger, as well as validating the status of the capacitive discharge circuit within the probe. This is a conceptual rendering of concentric BaDGE^{®} DNA electrotransfer probe. Features include an inner core DNA delivery needle which has a pre-determined length of conductive surface with an insulation layer extending to the internal contact point within the hub. Set back from the outer insulated surface is the outer conductive needle sheath of length and surface area that contributes jointly with the distal electrode surface and intervening insulated region, in creating the shaped electric field for DNA delivery. Proximal to the conducting region of the outer sheath, the surface is insulated except for the contact point within the hub. The hub consists of three ports - two of which are used to charge the capacitor, while the third port provides a sensor contact to indicate charging, all enabled by inserting the probe into a remote charging station. In another embodiment, the monitoring circuit may assess current flow between the two charging contacts and detect insertion using this measure. An example of a conceptual rendering of insertion of a probe 1510 into a charging port 1530 charging station 1520 is shown in figures 15a and 15b. Not shown is the protective needle cover which in one embodiment would be in place until just prior to delivery of the DNA. The hub contains an integrated capacitor which is discharged by closure of an internal switch actioned by deflection of a pin element within the hub that contacts the fully or substantially fully depressed plunger of the syringe barrel that would contain the DNA solution and be attached to the hub of the DNA delivery probe. Figure 16 provides further conceptual drawings of a syringe body 1610 with integrated needle array and discharge capacitor 1630. This example also includes a contact rod 1640 to cause switch actuation to discharge the capacitor via the array, when the syringe plunger reached the end or near end of its travel.

In some embodiments the electric discharge circuit comprises a single capacitor. This can have the advantage of being very small and easily embedded within a conventional device structure, such as a syringe. An example of the capacitor that will be integrated into the SCDGET needle hub is: small 2.2uF 450V Multilayer Ceramic Chip (MLCC) capacitors in it. A known MLCC commercially available component at the time of filing has dimensions of 5.7 x 5.0 x 2.5 mm.

An embodiment of the proposed system incorporates a disposable concentric needle-type DNA delivery probe that looks and feels like a conventional hypodermic needle, is compatible with conventional syringes and once touched against a remote charging unit, makes SCD-BaDGE^{®} DNA delivery virtually indistinguishable from a routine vaccination procedure. Already revolutionary, our SCD-BaDGE^{®} DNA delivery system is so efficient that a single electric pulse arising from discharge of a small capacitor integrated into the hub of the needle-like DNA delivery probe provides 100% reliable gene expression in muscle. However, the use of this technology is not limited to muscle tissue, for example this system may also be used to transfect brain tissue, retina or other tissue types.

Alternative embodiments not currently illustrated in the drawings are also contemplated. For example, an embodiment may include metal snap-action type contacts to form an electrical connection. Another embodiment may include a magnet and reed switch to form an electrical connection when the magnet attached to the syringe plunger comes in close proximity of the reed switch when the syringe plunger reached the end or near end of its travel. Another embodiment may include (1) metal snap-action switch contacts and (2) a dual-shaft syringe wherein the second shaft of the syringe is used to house the electrical switch, actuable by operation of the plunger. For example, switch contacts may be included at the bottom of a second syringe shaft.

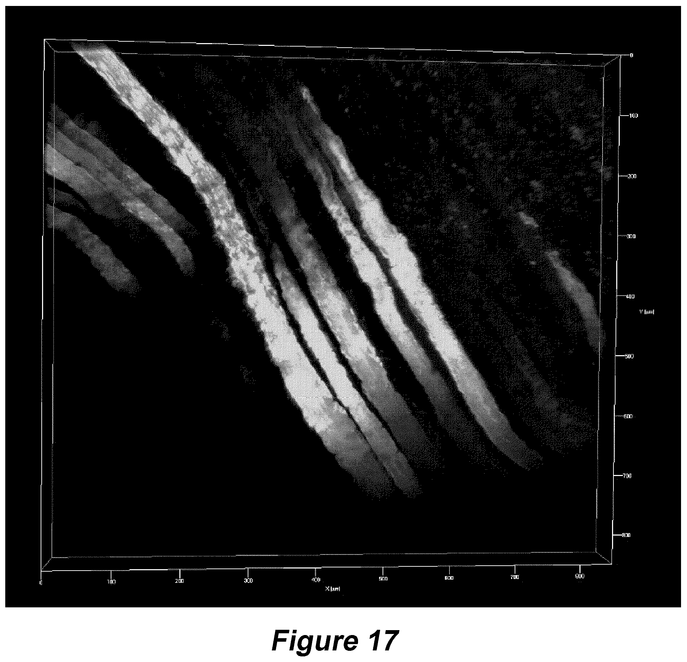

Figure 17 is an image demonstrating the utility of a prototype SCD-BaDGE *in vivo* application, the image showing expression of light activatable ChrimsonR channelrhodopsin ion channel protein - TD tomato fusion reporter protein in mouse soleus muscle fibres. This demonstrates use of single capacitance discharge BaDGE for a mouse optogenetics application. Expression of light activatable ChrimsonR channelrhodopsin ion channel protein **-** TD tomato fusion reporter protein in mouse soleus muscle fibres was imaged 4 days after single capacitive discharge electrotransfer (SCD-BaDGE^{®}) of the naked plasmid DNA encoding this protein under a cytomegalovirus promoter. DNA delivery parameters: 1 µg/µl DNA in iso-osmotic sucrose-based carrier solution, the BaDGE^{®} DNA delivery probe included a 2.2 µF capacitor charged to 250 V. The image is a 3D reconstruction of a confocal laser scanning image stack acquired using 561 nm excitation and 567 - 633 nm emission. The image shown in Figure 17 shows individual muscle fibre expression as a result of this *in vivo* gene electrotransfer.

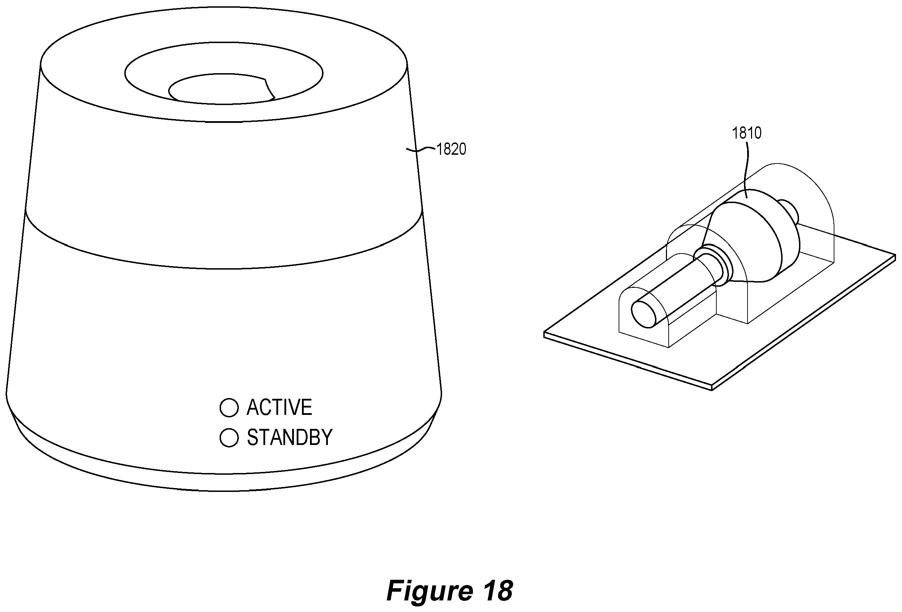

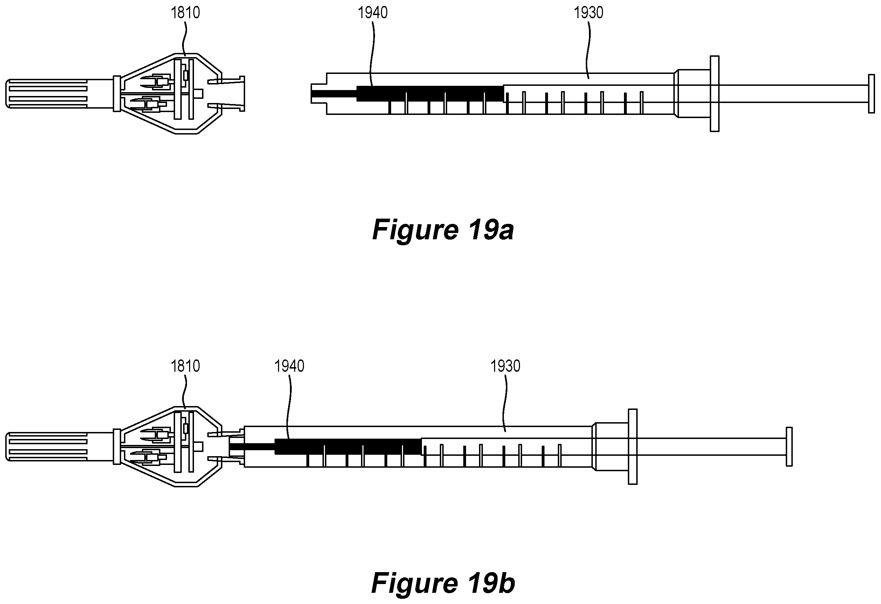

Figure 18 illustrates an example of one possible system embodiment, comprising electrotransfer modules 1810, each connectable to a syringe (not shown), and a charging unit 1820. Each electrotransfer module 1810 includes a capacitive discharge circuit, which is chargeable using the charging unit 1820. Figure 19a shows an example of the electrotransfer module, which is configured to be compatible with syringe 1930. For example, the electrotransfer module 1810 may be Luer-lock compatible to engage with a conventional Luer syringe. Figure 19b illustrates the electrotransfer module 1810 connected to the syringe 1930. This may be advantageous as this allows the syringe to be pre-loaded with the desired therapeutic fluid 1940 or this may be loaded on site. It should be appreciated that the electrotransfer module may be utilised for electro-stimulated delivery of a plurality of different therapeutics, the therapeutic substance may have short shelf life or special handling conditions. Thus, it may be practical to supply the therapeutic substance separately from the electrotransfer modules, which being devices do not have similar shelf life restrictions. Having the electrotransfer modules as separate devices from the therapeutic delivery syringes may also provide greater flexibility and ability to adapt to changing therapeutics. For example, one batch of electrotransfer modules may be purchased in bulk for a clinical trial comparing two or more therapeutics (and controls) or for a clinical practice treating numerous conditions, each using a different therapeutic.

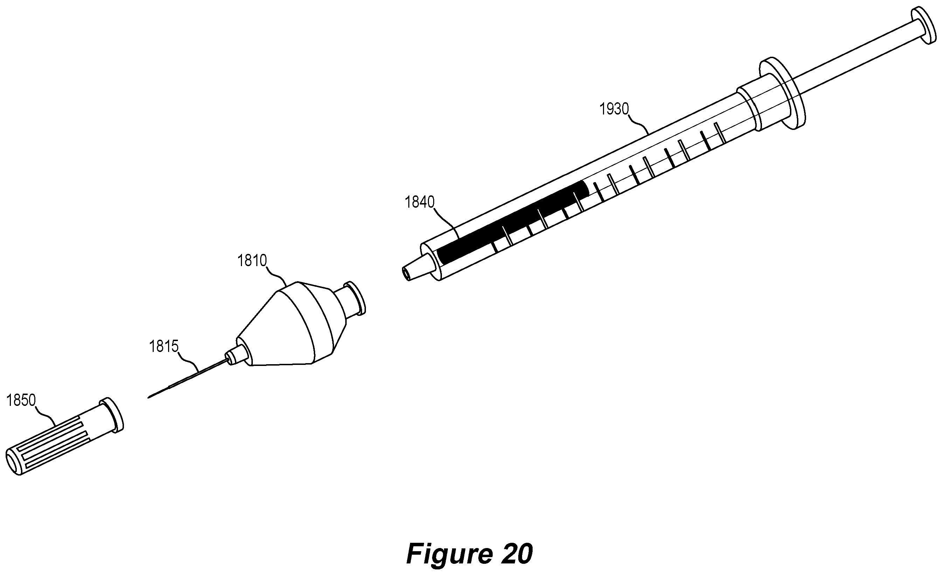

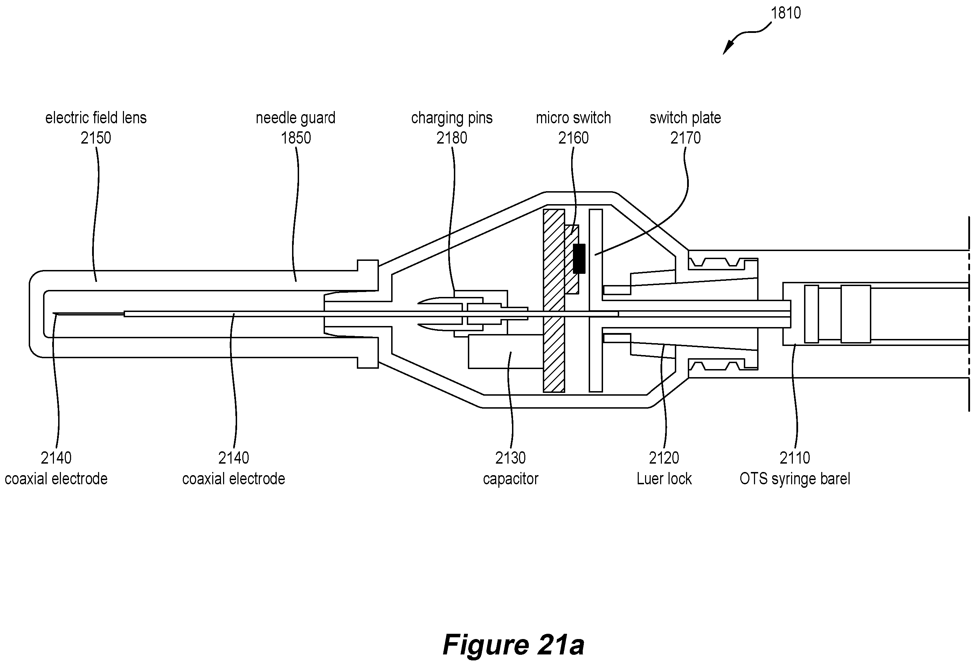

Figure 20 shows an exploded view of a syringe 1930 and electrotransfer module 1810. The electrotransfer module has a needle array 1815 (as described in any of the embodiments above) which can ordinarily be covered using a needle cover 1850 for safe handling. Figure 21a shows an example of an embodiment of an electrotransfer module 1810 in further detail. The electrotransfer module 1810 has a housing with a neck portion 2120 at one end connectable to a syringe and the needle array projecting from the opposite end with a lumen extending through the device to enable delivery of fluid from a connected syringe. The syringe may be an off the shelf (OTS) syringe 2110, in this example a Luer-lock type syringe, and the housing is configured to be compatible with the Luer lock 2120. However other attachments may able be used. The needle array comprises two coaxial electrodes with insulation between, the first, interior, electrode is longer than the second, exterior, electrode and the insulation extends along part of the first electrode beyond the end of the second electrode, to create an insulated gap 2150 between the two electrodes. The length of this gap 2150 influences the electric field generated by the capacitive discharge and thereby the area targeted for electric field mediated cell transfection (electrotransfer). Thus the gap may also be referred to as an electric field lens 2150 due to its impact on focussing the electric field gradients.

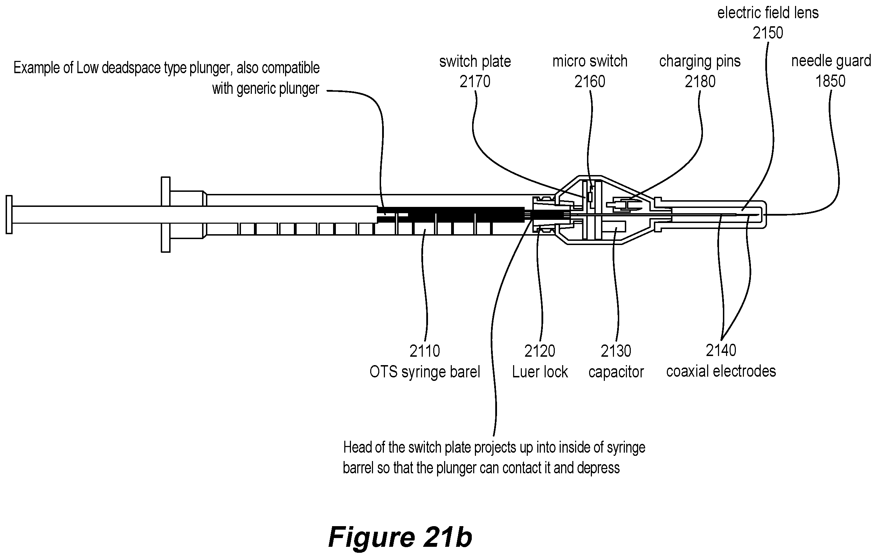

The housing houses a capacitive discharge circuit and the mechanism for actuating the capacitive discharge. The capacitive discharge circuit is carried on a printed circuit board and includes a capacitor 2130 to store charge for delivery, a switch and is electrically connected to the electrodes of the needle array. The circuit also includes charging pins 2180 or another means for enabling an external electrical connection to charge the capacitor. In this embodiment the switch is a microswitch 2160 and the switch actuator is a switch plate 2170 configured to be acted on by the syringe plunger toward the end of the plunger's travel to move the switch plate 2170 to actuate the microswitch 2160 and cause discharge of the stored charge from the capacitor. In this embodiment the switch plate 2170 has a planar section configured to engage with the microswitch and a post extending through the neck portion 2120 of the housing to be engageable with the syringe plunger, and including a lumen connected to the needle array to establish a fluid communication channel through from the syringe to the needle array for delivering fluid to tissue. In this embodiment the switch plate 2170 is biased (or otherwise initially held mechanically) to an open (or disengaged) position. Actuation of the syringe plunger will initially force fluid through the lumen, and eventually the plunger will contact the post and continued travel of the plunger cause corresponding movement of the switch plate toward the microswitch to actuate the microswitch, and trigger the capacitive discharge via the electrode array. Figure 21b shows an alternative embodiment of the electrotransfer module, designed to operate with low dead space type syringes. This embodiment has a variation in the switch plate, with the post being slightly shorter due to a different syringe plunger shape.

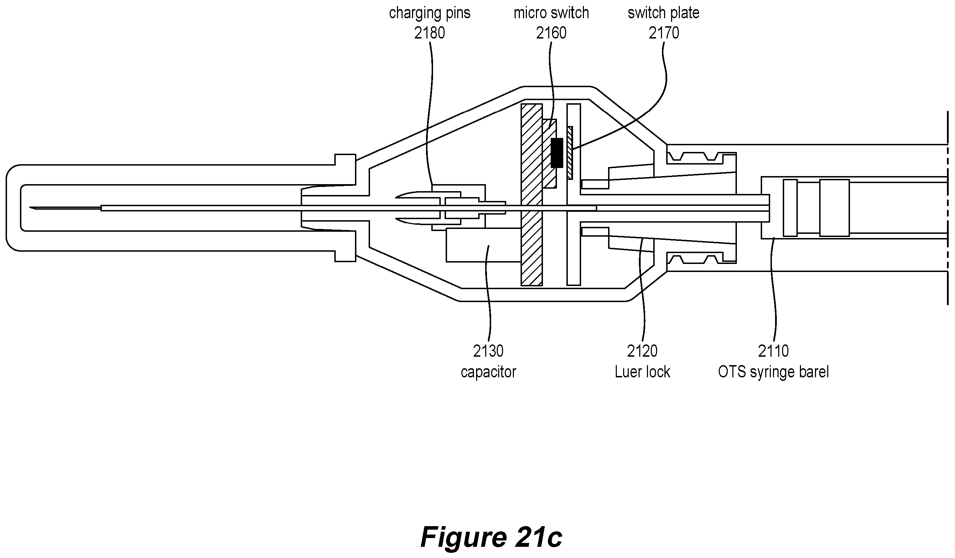

In the embodiment shown the capacitive discharge switch is a microswitch actuated by a switch plate, which is mechanically moved to actuate the switch by the syringe plunger. Other types of switches and actuators are also envisaged within the embodiments of the system. For example, the switch could be a magnet in the switch foot plate to activate a micro magnetic reed switch or a piston that has a conductive (metal) ring which is driven down to close the discharge circuit. Figure 21c is an example of Reed relay and magnetic actuation of capacitive charge delivery occurs when magnet embedded on switch plate 2175 comes in close proximity to reed relay 2165, causing the switch within the relay 2165 to close its contacts, allowing the capacitor to discharge through the needle electrodes.

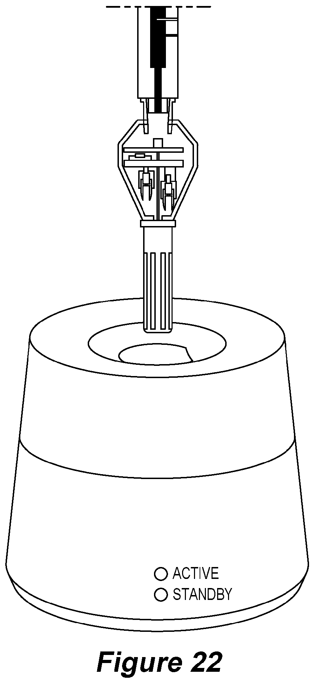

Figure 22 illustrates the electrotransfer module (attached to a therapeutic fluid filled syringe) being inserted into a charging unit to charge the capacitive discharge circuit. The charging unit is connected to a power supply. When the electrotransfer module is inserted, an electrical connection is made to the capacitive discharge circuit via charging pins. A light on the charging device and/or an audible alert can indicate when the capacitor is fully charged. The device can then be removed and the patient treated.

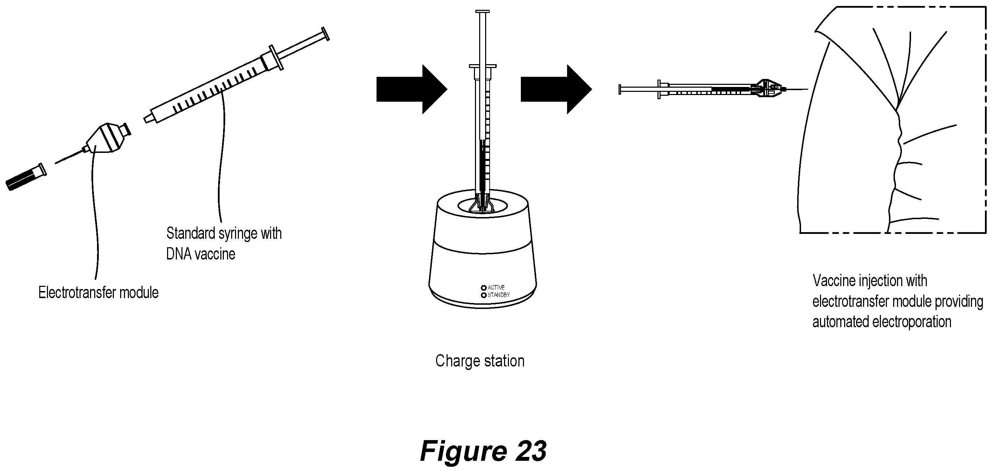

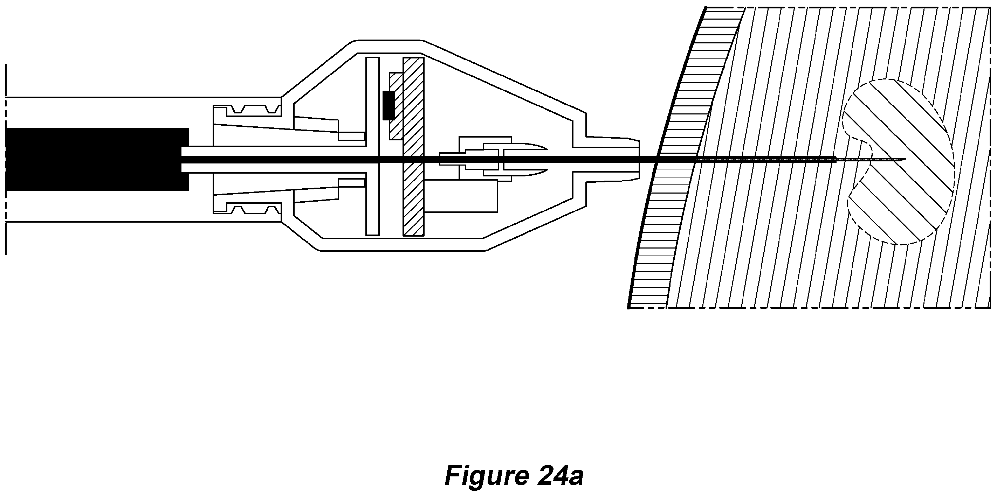

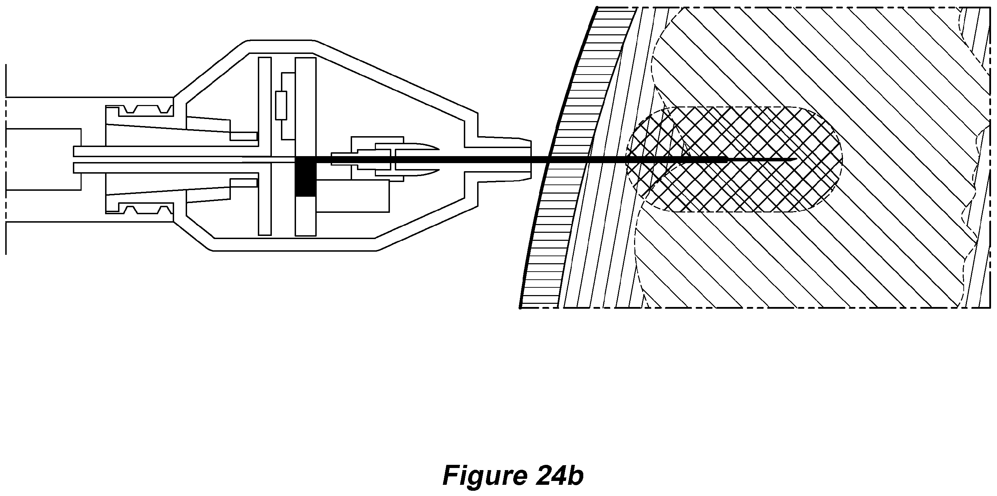

As is shown in the process illustration of Figure 23 the clinician first assembles the syringe and electrotransfer module, then charges the capacitive discharge circuit, and then delivers the treatment to a patient similarly to any typical inoculation injection. Figure 24a illustrates the first stage of the inoculation where the needle is inserted into the patient's arm and the therapeutic fluid delivered to the tissue around the needle array as the syringe plunger is depressed. As the plunger approaches the end of its travel, it actuates the capacitive discharge circuit switch, this causes charge from the capacitor to discharge via the electrodes of the needle array and create electric field gradients in the tissue surrounding the array which stimulates cell transfection.

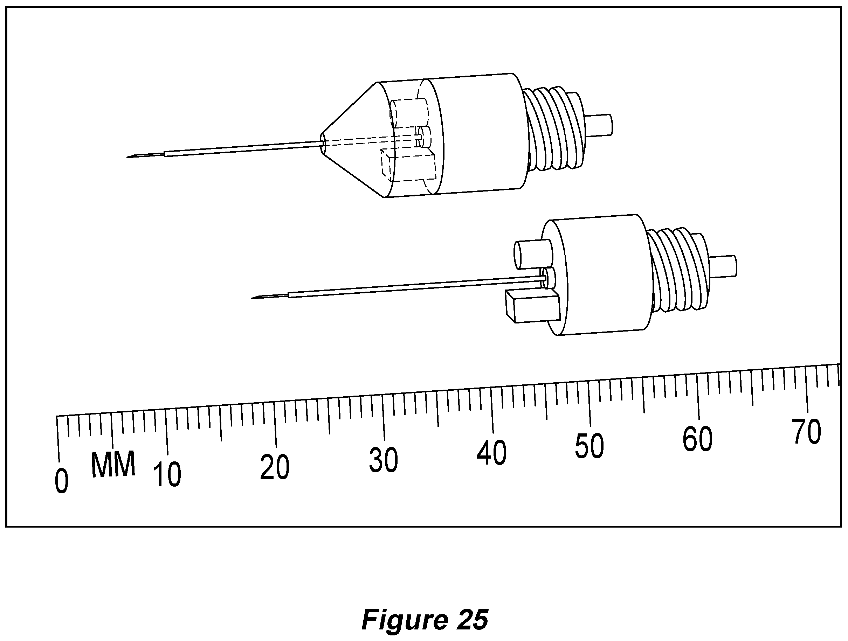

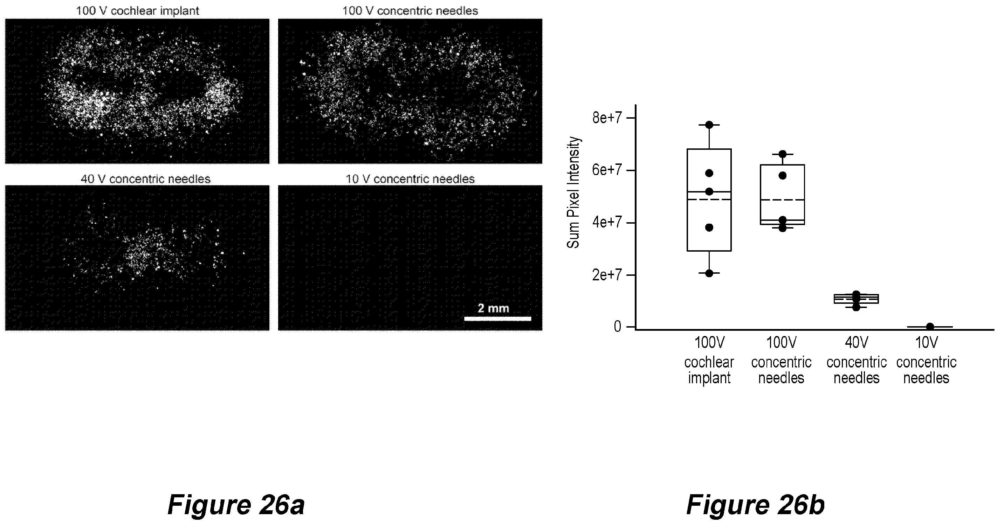

Figure 25 is a photograph of a prototype embodiment of an electrotransfer module. Figures 26a and 26b show results comparing DNA electrotransfer using an 8 node bionic array, and a concentric stainless steel electrode array using HEK293 monolayers. Fluorescence of the mCherry reporter gene was captured (shown in figure 26a) 4 days after BaDGE^{®} to HEK293 cell monolayers using a single capacitive discharge from a 2.2 µF capacitor charged to either 100 V, 40 V or 10 V. The electrodes used for gene delivery were either a cochlear implant 8-electrode array (Pt/Ir electrodes 400 µm length and diameter and spacing, in 'tandem' configuration(+ + + + - - - -), against concentric stainless needle electrodes (34 G inner and 28 G outer needles) with active electrodes 2 mm in length and a 1 mm gap between electrodes. n = 5 coverslips for each experimental protocol.

Cells were seeded onto 18 mm coverslips approximately 24 hrs before gene transfer, which was undertaken when the cells were roughly 50% confluent on the coverslips. For BaDGE^{®} 20 ul of DNA at 2 ug/ul (CMVp-mCherry plasmid) suspended in pH neutral 10% sucrose solution was applied to the cell coated coverslips after the aligning the electrode above the cells. Figure 26a is a set of images showing the results of electrotransfer using a single capacitive discharge comparing results for electroporation using a cochlear implant array and the prototype electrotransfer module. The images show fluorescence evidencing transfection of HEK293 cell monolayers for:
1. An 8 electrode cochlear electrode array with four adjacent electrodes driven as an anode array and the remaining four adjacent electrodes driven as a cathode array, also known as a tandem array configuration (+ + + + - - - -), driven using a 100 V charge to a 2.2 µF capacitor, single discharge. This is an electrode configuration proven previously by the inventors to be effective for electrotransfer using sequences of pulses and was used for initial proof of efficacy of electrotransfer using single capacitive pulse discharge, and so is used for comparison here with the concentric needle array prototype testing.
2. The prototype concentric needle array after electrotransfer driving the needle electrode array using a 2.2 µF capacitor, single discharge, of 100V. This shows a similar transfection field to the cochlear electrode embodiment driven using the same pulse.
3. The prototype concentric needle array after electrotransfer driving the needle electrode array using a 2.2 µF capacitor, single discharge, of 40V. This shows a lower intensity transfection response, which is not surprising given the lower charge.
4. The prototype concentric needle array after electrotransfer driving the needle electrode array using a 2.2 µF capacitor, single discharge, of 10V. This shows a minimal transfection response.

Figure 26b is a boxplot graph of the results, illustrated in figure 26a. These results show that efficacy for DNA delivery to HEK293 cell monolayers transfects cells in an equivalent area when using an 8 electrode array in tandem configuration (+ + + + - - - -), and when using the prototype electrotransfer module ('BaDGE-Inoculator') with an electrode array comprising stainless steel concentric needles, both driven by a 100 V charge to a 2.2 µF capacitor single discharge. (P = 0.00016, t-test comparing 100 V and 40 V concentric electrodes; P = 0.008, 40V vs 10 V , Mann-Whitney rank sum test). This also shows that for 40V less cells are transfected with the transfection focusses around the region in the middle region, where the electric filed gradients focussed around the gap between the electrodes, the electric field lens. In the example of the using 10V charging there is minimal transfection indicating this voltage provides insufficient electric field strength for transfection with this combination of electrotransfer module prototype and therapeutic.

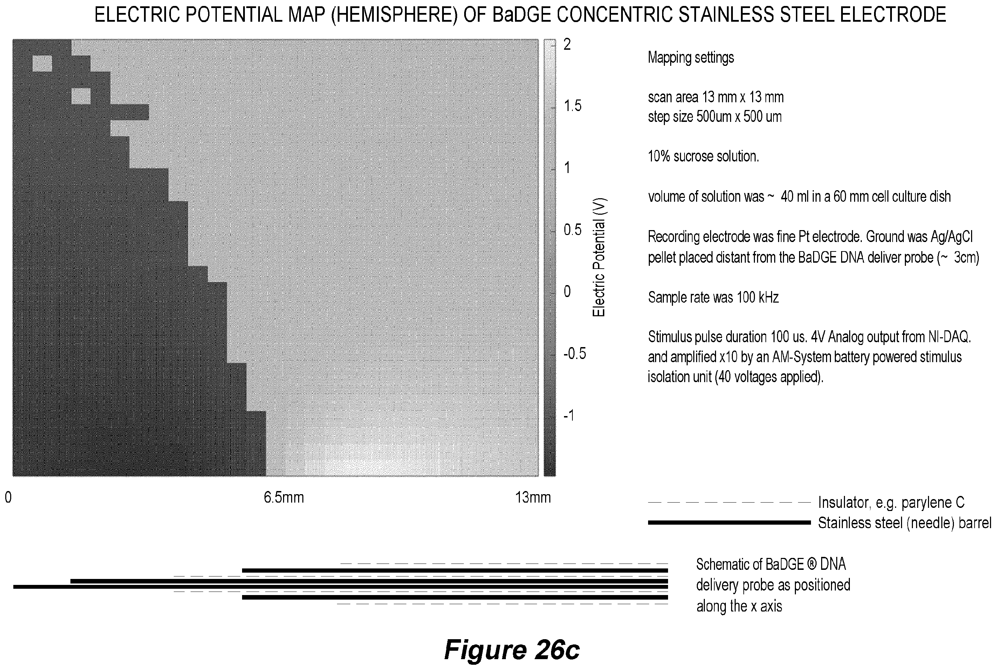

Figure 26c shows an electric potential map (hemisphere) of BaDGE concentric stainless steel electrode array, a schematic (cross section) of the needle electrode array is also illustrated below and aligned with the potential map. This matches the field used to achieve HEK293 cell transfection at 40V single capacitive discharge as described above in relation to figure 26a.

An extension of the studies discussed above was undertaken with a second cohort of mice to show reliable Luciferase expression using the BaDGE^{®}-Inoculator (stainless steel concentric needles), with single capacitive discharge at either 40V or 100 V to drive DNA experssion by the mouse hindlimb muscle fibres - 1 µg/µl luciferase reporter plasmid DNA in 10% sucrose solution, 50 µl total volume.

Figure 27 illustrates results of temporal tracking of BaDGE^{®}-Inoculator - mediated gene expression. (A) Representative bioluminescence image demonstrating the spread of luciferase activity three days following intramuscular BaDGE^{®} mediated luciferase gene (Luc) transfer to the mouse gastrocnemius muscle. (B) Representative trace of the emitted photons per second in a ROI placed over a hind limb, measured in one-minute intervals following i.p. luciferin injection. (C) Time course graphing bioluminescence measurements at peak (red arrow in B) one, three and seven days following BaDGE^{®} mediated transfer of the luciferase encoding plasmid using a single capacitive discharge at either 40V or 100 V charging of a 2.2 µF capacitor (alternative legs). DNA plasmid reporter delivery was pMK175-CAGp-Luc (50 µl; 1 µg/µl; carrier was 10% sucrose @ pH7.4; n = 5 gastrocnemius muscle inoculations for each capacitor charging voltage; mean ± s.e.m.).

The methods applied to obtain these results are as follows:
All procedures followed procedures approved by the UNSW Animal care and ethics committee (ACEC 18_160a). The animals were placed in an induction chamber and anaesthesia induced using 3% isoflurane with oxygen at a flow rate of flow rate of 1-2 L/min. Once anaesthetised, the animal was transferred to a heated animal bed in the prone or supine position while anaesthesia was maintained by delivery of isoflurane anaesthetic via a nose cone at 2 % Isoflurane with oxygen at 1-2 L/min. Eye ointment was applied. The body temperature was maintained, and the oxygen saturation monitored with a MouseStat PhysioSuite (Kent Scientific). Hind limbs and lower back of the animal was shaved and disinfected with an ethanol swab.

BaDGE^{®} - mediated transfer of the luciferase encoding plasmid used a single capacitive discharge at either 40V or 100 V charging of a 2.2 µF capacitor (alternative legs). DNA plasmid reporter delivery was pMK175-CAGp-Luc (50 µl; 1 µg/µl; carrier was 10% sucrose @ pH7.4; n = 5 gastrocnemius muscle inoculations for each capacitor charging voltage; The animals were allowed to recover before returning them to their home cage.

Bioluminescence imaging was conducted repeatedly on a Spectrum computed tomography scanner (IVIS^{®} SpectrumCT, Perkin Elmer). For this procedure anaesthesia was induced in an induction chamber with 3% isoflurane with oxygen as specified above. Once anaesthetised, eye ointment was applied, and mice were given an intraperitoneal injection of luciferin (150 mg/kg). At this dose luciferin is an innocuous substrate that emits photons upon reaction with Luciferase expressed from the pMK175-CAGp-Luc plasmid delivered by BaDGE^{®}. The animals were then transferred to a heated animal bed in the prone or supine position while anaesthesia was maintained by delivery of isoflurane anaesthetic via a nose cone at 2 % isoflurane in the IVIS^{®} SpectrumCT. Bioluminescence 2720 was imaged in one-minute intervals until a consistent drop of radiance (photons emitted per second in a defined region of interest (ROI) 2710 placed over each hind limb) was detected over 5 consecutive minutes. The animals were then allowed to recover before returning them to their home cage. Peak luminescence 2730 was determined as maximal radiance in a given ROI.

Plotting the peak luminescence over time (bottom graph in Figure 27), with the first measurement taken 24 hours post electrotransfer, demonstrates reliable Luciferase expression, increasing over the first 72 hours. Measurements taken 7 days post electrotransfer show similar luminescence measured for the mice having electrotransfer stimulated using 40V and 100V to drive DNA uptake by the mouse hindlimb muscle fibres.

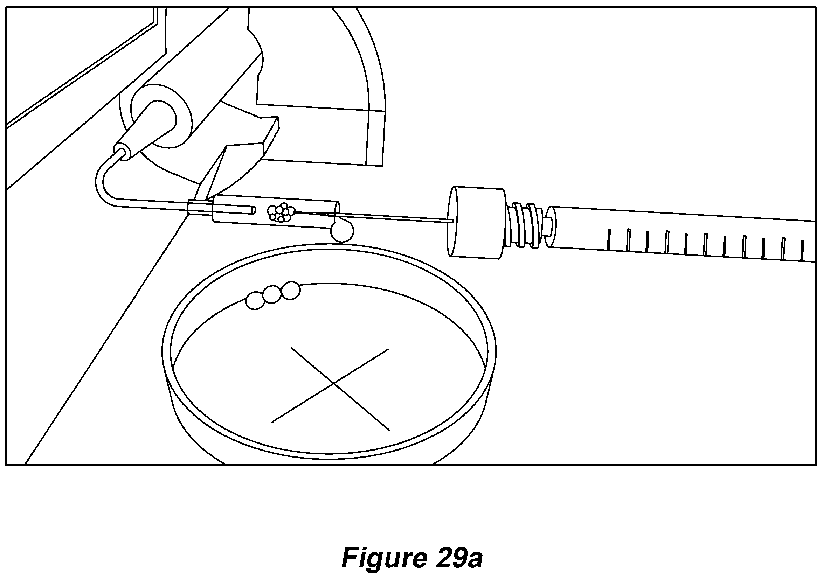

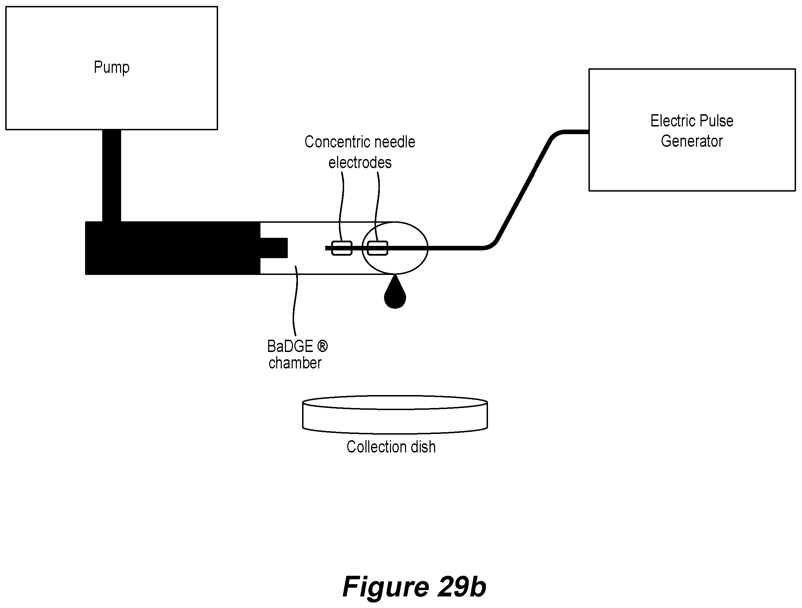

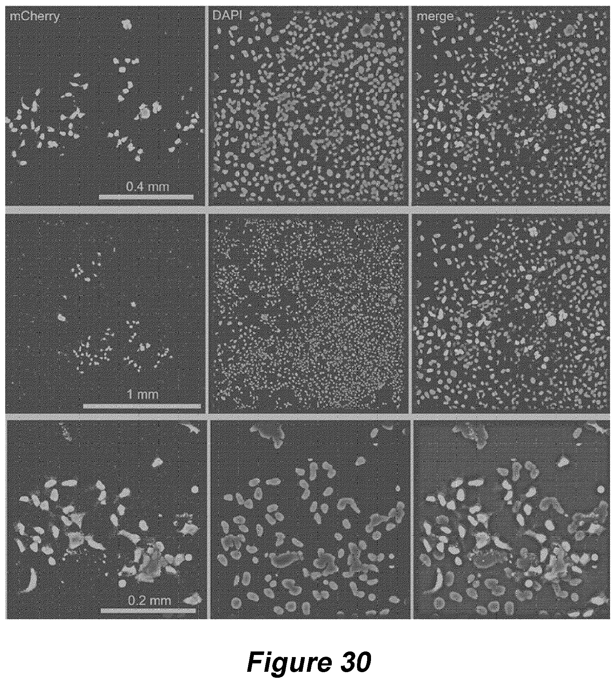

An alternative application of the disclosed electrotransfer technology is in DNA treatment for in vitro cell transfection for autologous gene therapy. Figure 29 illustrates a prototype test embodiment for high throughput needle electrode stimulates DNA electrotransfer in cell suspensions for autologous gene therapy and in vitro cell transfection. An example of a process for in vitro continuous cell transfection with naked DNA via needle electrode and capacitive discharge DNA electrotransfer is configured to capture cells from Culture (in vitro) or from the body (tissue cell suspension after digestion, or from blood, and resuspend in DNA carrier solution (10% sucrose, with DNA at typically > 0.1 µg/µl - 10 µg/µl). This suspension is pumped through a reaction tube via a syringe pump so that the suspended cells migrate past the needle electrode DNA delivery probe (~ 5 mm length) with a passage time (depending upon tube diameter) that ensures an electroporation pulse is delivered at least twice across that distance. Typical electroporation protocol is a 100 V exponential decay via a 2.2 µF capacitor at 2 Hz. Cells in solution then transfer to culture medium for culture, or re-pelleting to be returned to the body (autologous cell gene therapy application). DNA electrotransfer is instantaneous. For in vitro readout - cells can be counted using FACS (Fluorescence-activated cell sorting) from ~ 10 hours post transfection. Figure 30 shows images of cells showing expression of an mCherry reporter plasmid DNA against the background of cells (DAPI nuclear stain) following continuous flow DNA electrotransfer.

In this cell flow experiment, media was removed and 1 ml of TryplE was added to a single 10 cm diameter dish with HEK293 cells roughtly 90 % confluent. After 3 minutes at 37oC 9 ml of 10% sucrose was added to the dish and cells were resuspended. Cells from half (5ml) of this suspension were pelleted at 800 rpm for 5 minutes. The 5 ml of sucrose solution was removed, and the cell pellet was resuspended in 0.5 ml of pH neutral 10% sucrose containing 1 ug/ul reporter plasmid DNA (pJLP17-CMVp-mCherry). The cell-DNA suspension was collected in a 5 ml syringe and attached to an infusion syringe pump set to deliver 1 ml every 6 minutes (250 µl over 90 seconds). The syringe delivered the cell-DNA solution around the concentric needle electrode (34 G inner and 28 G outer needles) with active electrodes 2 mm in length and a 1 mm gap between electrodes (total array length 5 mm). Capacitive discharge modelled pulses were delivered to the electrode via a Digitimer DS5 constant-current stimulator controlled by a microprocessor to model a 2.2 µF capacitor discharge. Pulses were delivered at 2 Hz at 50 mA/120 V with a 10 ms decay constant. Cells were collected after passing the electrode in a 5 cm culture dish with fresh media and incubated at 37oC 5% CO2 for 6 days.

6 days after BaDGE^{®} DNA delivery cells were fixed in 4% paraformaldehyde for 20 minutes and counter-stained with DAPI to label all cell nuclei. mCherry reporter fluorescence was imaged using a 561 nm DPSS laser and DAPI using a 790 nm Titanium Saphire multi-photon laser. The results are illustrated in Figure 30.

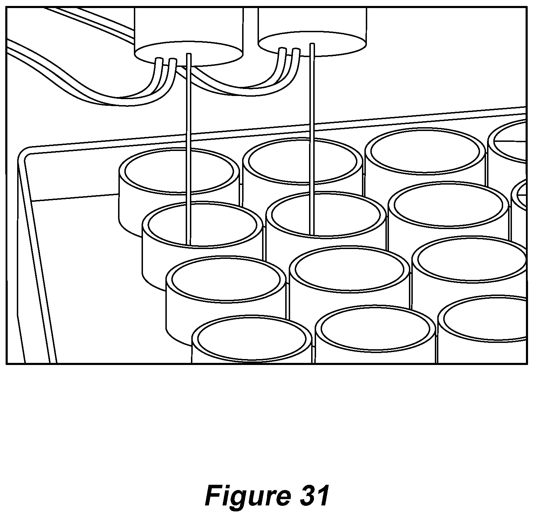

Figure 31 illustrates another example of the electrotransfer system being utilised in in vitro cell therapies. The described DNA delivery probe in stainless steel concentric needle configuration (or Pt/Ir or other electrode materials), is ideal for high throughput DNA electrotransfer into cells either adherent to, or suspended in multi-well plate configurations. The pulse parameters, such as capacitive discharge, or conventional monopolar square wave pulses, or other pulse-parameters in either constant current, or constant voltage , or complex waveform profiles, to provide high efficient DNA electrotransfer with the advantage for (robotic) multi-well high throughput in providing highly efficient cell transfection with naked (plasmid) DNA and not requiring any current return path hardwired or separately inserted into the transfection wells, which would be neither as efficient, not as convenient for rapid deployment between wells while minimizing cross-well contamination / carry-over. Cells can be cultured in situ, or extracted from the wells immediately after DNA electrotransfer.

Individual needle electrode DNA probes can be provided with different pulse / pulsetrain parameters to provide rapid optimization of DNA transfection parameters, to achieve the most efficient DNA (or other molecular type) delivery to cells

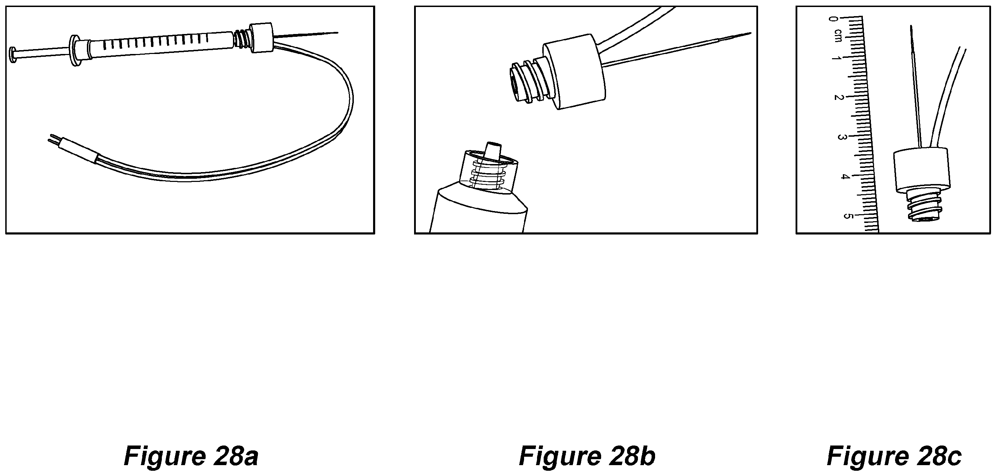

In an alternative embodiment, for example a minimum viable product (MVP) - to enable mass production with minimum time lag - an embodiment is contemplated where a disposable needle array is utilised that is connectable to a syringe and an external charge delivery circuit. For example, a needle array may be provided (in a sterilised package) with an external, wired electrical connector to connect the needle array to the pulse delivery circuit. An example of a prototype of this embodiment is illustrated in figures 28a-c.

Producing the needle arrays for this embodiment may involve coating the needles using a Parylene C coating with the electrode surfaces masked from this insulator, the inner needle electrode would be pushed into a hub so that the upper end enters a connector cone which would scrape off the insulator and continuity could be checked, then the outer needle would be pushed over the inner needle and be inserted into a larger diameter cone. The cone being configured within the needle hub, which in turn provides a standard connection to a syringe such that the inner needle electrode lumen is in fluid communication with the syringe reservoir for delivery of the DNA vaccine fluid. Leads from both (cone) connectors would run to an external wire which would have a connector on the end.

The pulse delivery circuit may be a known pulse delivery device (suitably programmed) or a simplified capacitive discharge unit, incorporating a capacitive discharge circuit, and charging circuit configured specifically for the required charge delivery. Effectively placing the capacitive discharge circuit and charging unit of an embodiment of the electrotransfer system, as discussed above, into one unit, connectable by wire to disposable needle arrays. The capacitive discharge pulse may be triggered manually by the clinician, for example using a manual switch, which may be a foot actuated. In this embodiment, the capacitor would be in the charging box, thus enabling reuse of the charging circuit. This has an advantage of requiring less charging circuits to be produced for mass deployment of electrotransfer DNA vaccine delivery than would be needed for the disposable electrotransfer modules discussed above. This also has an advantage for scale up production of flexibility of choice in capacitor suppliers (for example to have a choice of suppliers and an order size of about 100,000, while the production model for the disposable BaDGE-inoculators could be up to 1 million per month (2 per second ....)). For example, in an embodiment the concentric needle array may be robotically manufactured with the concentric insulated needles inserted into a hub under high throughput manufacture. The hub can be configured to connect to an off the shelf syringe (i.e. Luer-lock syringe). The hub may be formed of insulating material (i.e. plastic) two concentric metallic (i.e. stainless steel) contact cones, with insulation between, arranged to each to provide an electrical connection between one of the needle electrodes and a lead for connection to an external charge delivery circuit. The needles may be coated (or part coated leaving the electrode tip portion clear) with an electrically insulating material (for example, parylene coated). The end that is going into the hub can then be inserted by a robotic armature, the first (small internal) needle passes furthest into the hub, through an aperture in a first contact cone to contact the smaller second contact cone nested inside the first cone. Press fitting scapes off the edge of insulation (for example 25 µm) and makes the contact, which can be confirmed by a continuity test from the armature to the lead wire soldered to the connector. The second needle is then inserted over the smaller inner needle and into the hub, it is a larger diameter and so makes contact with the larger diameter contact cone to form an electrical connection. Heat setting or other mechanism (i.e. adhesive or pressure) may be used to secure the needles in place.

The needle array unit would be sterile packed, including wire and connector, and disposable; the controller (for example, 2 needed per clinic) would enable tens of thousands of cycles. Thus, extreme production scale up is required for the needle electrode array component only to meet pandemic emergency demand. This can enable manufacture from readily sourced raw materials. The needles used for the concentric needle electrode array can use standard gauges, readily sourced from multiple suppliers.

It should be appreciated that this naked DNA delivery platform technology offers significant advantages in circumstances such as the unprecedented need for mass vaccination to help stem the spread of COVID-19. Where any solution will need a rapid path to large scale deployment. DNA vaccines work by getting the body to produce antibodies without directly injecting actual virus protein fragments. Last year a DNA vaccine designed as a treatment to prevent the MERS-coronavirus infection was found to produce clinically significant levels of antibody production in Phase 1 clinical trials. The COVID-19 virus is analogous in structure to MERS and clinical trials are currently underway in the USA with an adaptation of the MERS vaccine to address COVID-19, expecting a similar performance to the MERS vaccine. In addition to this MERS-COVID-19 DNA vaccine derivative, several other groups around the world are also developing DNA vaccines against COVID-19. As soon as any group demonstrates efficacy, the DNA sequence will become generally available for anyone to manufacture. One clear advantage of these plasmid DNA vaccines over serum vaccines is the DNA chemical sequence can be distributed electronically and produced anywhere. Being naked DNA, the COVID-19 DNA vaccine requires an electroporation delivery system to administer (gene transfection occurs using brief electric pulses). However, current commercial delivery units are both expensive and complex, making them difficult to scale to the high-volume production and low cost required for mass inoculation in this pandemic outbreak.

The applicant's single pulse electroporation technology designed for DNA vaccine delivery described herein has advantages of being is more efficient, offers a lower production cost, uses less DNA (freeing up more DNA vaccine doses) than current clinical electroporators, and is painless. Critically, this technology is highly scalable for mass manufacturing, with minimal components, facilitating rapid deployment. As soon as a DNA vaccine for COVID-19 is identified and produced, the disclosed electrotransfer technology can be utilised to address the delivery needs.

Advantages of embodiments of the presently described system include:
1. This enables disposable DNA gene electrotransfer device
2. DNA gene delivery system suitable for low-cost high-volume manufacture
3. Set charge delivery is inherently safe
4. Stand-alone charging station suitable for field operation
5. A complete 'game changer' for DNA vaccine applications, but also more broadly applicable to achieving a wide range of DNA therapeutics applications
6. Separates the DNA electrotransfer process from the power supply and lowers the cost of the technology Immediately suitable for development as a medical device / drug combination for tissue targets for DNA therapeutics - e.g. DNA vaccines, solid tumor oncology targets (melanoma, postrate cancer), nerve and muscle repair (reinnervation), neuromodulation of targeted brain areas. Field applications for trauma treatment.

It will be understood to persons skilled in the art of the invention that many modifications may be made without departing from the spirit and scope of the invention.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

It is to be understood that, if any prior art publication is referred to herein, such reference does not constitute an admission that the publication forms a part of the common general knowledge in the art, in Australia or any other country.

## Claims

1. A needle electrode array comprising:
a first needle which also acts as a first electrode;
a first concentric insulator sheathing the first needle to a predetermined first distance (L1) from the needle tip,
a concentric second electrode sheathing the first concentric insulator to a distance (L2) from a tipward end of the first concentric insulator, and
a second concentric insulator sheathing the second electrode to a distance (L3) from a tipward end of the second electrode, and
**characterised by**:
the first needle and second concentric electrode being formed of conductive material and configured for electrical connection to positive and negative terminals of a pulse delivery circuit,
the exposed length L1 of the first needle operates as a first electrode of a linear array, and the exposed length L3 of the second concentric electrode acts as the second electrode of a linear array, and
the respective lengths of the first and second electrodes, and exposed length of the first insulating portion therebetween determines the pattern of electric field gradients generated adjacent the needle array when an electric pulse is applied to drive one electrode as an anode and the other electrode as a cathode.

2. The needle electrode as claimed in any one of the preceding claims, wherein the first needle has a hollow lumen through which therapeutic solution can be delivered.

3. The needle electrode as claimed in claim 2, wherein the therapeutic solution is delivered to tissue from an aperture in the tip of the first needle.

4. The needle electrode as claimed in claim 2, wherein the needle array includes apertures in the array distal from the tip whereby therapeutic solution is delivered to tissue adjacent the needle array.

5. The needle electrode as claimed in any one of the preceding claims, wherein the needle tip is bevelled.

6. The needle electrode as claimed in any one of the preceding claims, wherein the needle array is mounted to a probe body an electrotransfer system probe, the needle array extending from the probe body.

7. The needle electrode as claimed in claim 6, wherein the needle array is connected via the probe body to a capacitive discharge circuit comprising capacitive charge storage configured to store a quantum of charge, and a switch actuatable to cause discharge of the stored quantum of charge through the needle electrode array.

8. The needle electrode as claimed in claim 7, wherein the capacitive charge storage is housed in the probe body.

9. The needle electrode as claimed in claim 7, wherein the capacitive charge storage is connected to the probe via a wired connection.

10. The needle electrode as claimed in any one of claims 7 to 9, further comprising a switch actuator carried on the probe body configured to enable actuation of the capacitive discharge circuit switch.

11. The needle electrode as claimed in any one of claims 7 to 10, wherein capacitive discharge circuit parameters are matched to resistivity parameters of a therapeutic solution for electrotransfer to minimise total charge delivered and optimise the discharge time constant for electrotransfer.

12. The needle electrode as claimed in claim 11, wherein capacitance of the capacitive discharge circuit is chosen based on a predicted total resistivity during discharge and a target accumulative charge, and the predicted total resistivity is based on the therapeutic solution resistivity.

13. The needle electrode as claimed in claim 11 or 12, wherein the matching is to achieve a discharge time constant of between 20 µs and 2 s.

14. The needle electrode as claimed in any one of claims 6 to 13 when dependent on claim 2, wherein the probe body further comprises a therapeutic reservoir to hold a volume of fluid in fluid communication with the lumen through the needle , and a therapeutic delivery actuator operable to cause the fluid to be forced from the therapeutic reservoir and through the lumen and be discharged from the needle.

15. The needle electrode as claimed in claim 14, wherein the probe body has a two part form, a first part incorporating the reservoir and therapeutic delivery actuator is provided by a syringe connectable to a second part comprising an electrotransfer module having a housing adapted to engage with the syringe, and support the needle electrode array projecting outward from the housing, and a fluid communication path is formed through the housing between the syringe reservoir and the lumen of the needle electrode.
